(19) Europäisches Patentamt
European Patent Office
Office européen des brevets

(11) Veröffentlichungsnummer: **0 020 859**
**A1**

# (12) EUROPÄISCHE PATENTANMELDUNG

(21) Anmeldenummer: **80101339.2**

(22) Anmeldetag: **14.03.80**

(51) Int. Cl.³: **C 07 C 103/365**, C 07 C 103/375, C 07 D 307/68, C 07 D 233/56, C 07 C 149/23, C 07 C 103/737, C 07 D 231/12, C 07 D 249/08, A 01 N 43/00, A 01 N 47/00, C 07 D 261/18

(30) Priorität: **21.03.79 DE 2910976**

(43) Veröffentlichungstag der Anmeldung: **07.01.81 Patentblatt 81/1**

(84) Benannte Vertragsstaaten: **AT BE CH DE FR GB IT NL SE**

(71) Anmelder: **BAYER AG, Zentralbereich Patente, Marken und Lizenzen, D-5090 Leverkusen 1, Bayerwerk (DE)**

(72) Erfinder: **Stetter, Jörg, Dr., Pahlkestrasse 3, D-5600 Wuppertal 1 (DE)**
Erfinder: **Lunkenheimer, Winfried, Dr., Bismarckstrasse 29, D-5600 Wuppertal 1 (DE)**
Erfinder: **Brandes, Wilhelm, Dr., Eichendorffstrasse 3, D-5653 Leichlingen (DE)**

(54) **Substituierte N-Allyl-acetanilide, Verfahren zu ihrer Herstellung sowie ihre Verwendung als Fungizide.**

(57) Die Erfindung betrifft neue substituierte N-Allyl-acetanilide, mehrere Verfahren zu ihrer Herstellung und ihre Verwendung als Fungizide.

Die Verbindungen der allgemeinen Formel

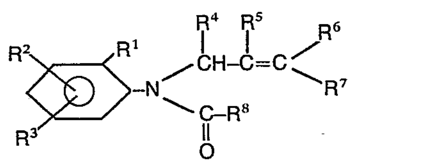

(I)

in welcher $R^1$ bis $R^8$ die in der Beschreibung angegebene Bedeutung besitzen, können z. B. erhalten werden, wenn man N-Allyl-anilide mit Säurechloriden oder -bromiden bzw. -anhydriden in Gegenwart eines Säurebindemittels umsetzt. Daneben gibt es noch andere Verfahren.

Die Wirkstoffe sind für den Gebrauch als Pflanzenschutzmittel geeignet und können mit besonders gutem Erfolg zur Bekämpfung vonOomyceten, z. B. gegen den Erreger der Kraut- und Braunfäule der Tomate und Kartoffel (Phytophthora infestans), eingesetzt werden.

EP 0 020 859 A1

BAYER AKTIENGESELLSCHAFT 5090 Leverkusen, Bayerwerk
Zentralbereich
Patente, Marken und Lizenzen Slr/kl
Ib

Substituierte N-Allyl-acetanilide, Verfahren zu ihrer Herstellung sowie ihre Verwendung als Fungizide

Die vorliegende Erfindung betrifft neue substituierte N-Allyl-acetanilide, mehrere Verfahren zu ihrer Herstellung und ihre Verwendung als Fungizide.

Es ist bereits bekannt geworden, daß Halogenacetanilide, wie beispielsweise N-Chloracetyl-N-(2,6-dialkylphenyl)-alanin- bzw. -glycin-alkylester mit gutem Erfolg zur Bekämpfung von pilzlichen Pflanzenkrankheiten eingesetzt werden können (vergleiche DT-OS 2 350 944 bzw. US-Patentschrift 3 780 090). Deren Wirkung ist jedoch, insbesondere bei niedrigen Aufwandmengen und -konzentrationen, insbesondere auch bei der Bekämpfung von Phytophthora -Arten nicht immer ganz befriedigend.

Es wurden nun die neuen substituierten N-Allyl-acetanilide der allgemeinen Formel

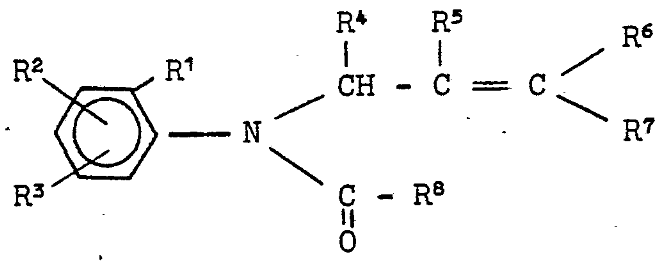

(I)

in welcher

$R^1$ für Wasserstoff, Alkyl, Halogen, Alkoxyalkyl oder Alkylthioalkyl steht,

$R^2$ für Wasserstoff, Alkyl, Alkoxyalkyl oder Alkylthioalkyl steht,

$R^3$ für Wasserstoff, Alkyl, Alkoxyalkyl oder Alkylthioalkyl steht,

$R^4$ für Wasserstoff, Alkyl oder Alkoxyalkyl steht,

$R^5$ für Wasserstoff, Alkyl, Halogen oder gegebenenfalls substituiertes Phenyl steht,

$R^6$ für Wasserstoff, Alkyl, Halogen oder gegebenenfalls substituiertes Phenyl steht,

$R^7$ für Wasserstoff, Alkyl, Halogen oder gegebenenfalls substituiertes Phenyl steht,

$R^8$ für Furyl, Tetrahydrofuryl, Thiophenyl, Tetrahydrothiophenyl; gegebenenfalls durch Alkyl substituiertes Isoxazolyl; gegebenenfalls durch Cyano oder Thiocyano substituiertes Alkyl, Alkenyl und Alkinyl; Dihalogenalkyl; sowie die Gruppierungen

$-CH_2-Az$, $-CH_2-OR^9$, $-CH_2-SR^9$, $-OR^9$, $-SR^9$, $-CH_2-OSO_2R^9$, $-COOR^9$ und $-CH_2-O-$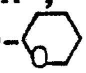
steht, wobei

$R^9$ für gegebenenfalls substituiertes Alkyl, Alkenyl, Alkinyl oder Alkoxyalkyl steht und

Az für Pyrazol-1-yl, 1,2,4-Triazol-1-yl und Imidazol-1-yl steht,

gefunden.

Die Verbindungen der Formel (I) können gegebenenfalls in den beiden geometrischen Isomeren cis und trans vorliegen; vorwiegend fallen sie als Gemische beider an. Alle Isomeren und die Gemische werden erfindungsgemäß beansprucht.

Weiterhin wurde gefunden, daß man die substituierten N-Allyl-acetanilide der Formel (I) erhält, wenn man

a) N-Allyl-aniline der Formel

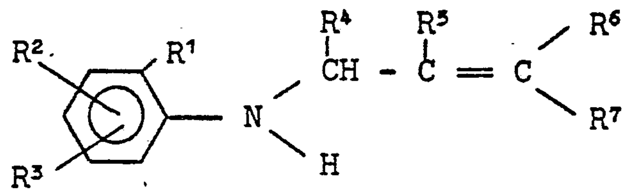 (II)

in welcher

$R^1$ bis $R^7$ die oben angegebene Bedeutung haben,

mit Säurechloriden oder -bromiden bzw. -anhydriden der Formeln

$$R^8 - \underset{\underset{O}{\|}}{C} - Cl(Br) \qquad (IIIa)$$

bzw.

$$(R^8 - \underset{\underset{O}{\|}}{C} - O)_2O \qquad (IIIb)$$

in welchen

$R^8$ die oben angegebene Bedeutung hat,

in Gegenwart eines Verdünnungsmittels und gegebenenfalls in Gegenwart eines Säurebindemittels umsetzt, oder

b) Anilide der Formel

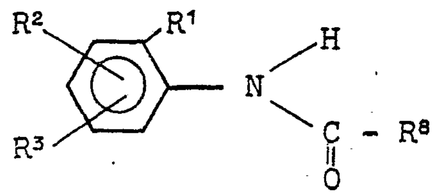

(IV)

in welcher

$R^1$, $R^2$, $R^3$ und $R^8$ die oben angegebene Bedeutung haben

mit Allylhalogeniden der Formel

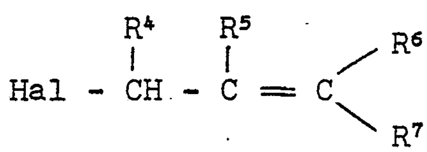

(V)

in welcher

$R^4$ bis $R^7$ die oben angegebene Bedeutung haben und

Hal für Chlor oder Brom steht,

in Gegenwart eines Säurebinders und gegebenenfalls in Gegenwart eines organischen Verdünnungsmittels oder in einem wässrig-organischen Zweiphasensystem in Gegenwart eines Phasentransferkatalysators umsetzt, oder

c) Aldehyde bzw. Ketone der Formel

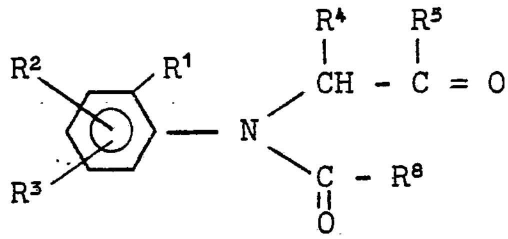

(VI)

in welcher

$R^1$ bis $R^5$ und $R^8$ die oben angegebene Bedeutung haben,

mit Phosphinalkylenen der Formel

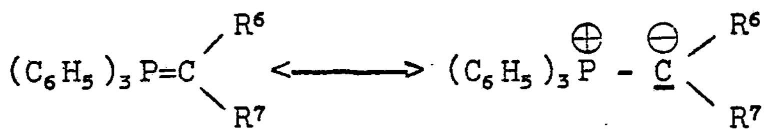

(VII)

in welcher

$R^6$ und $R^7$ die oben angegebene Bedeutung haben,

gegebenenfalls in Gegenwart eines Verdünnungsmittels umsetzt.

Einzelne Verbindungen der Formel (I) können auch erhalten werden, indem man

d) N-Propargyl-acetanilide der Formel

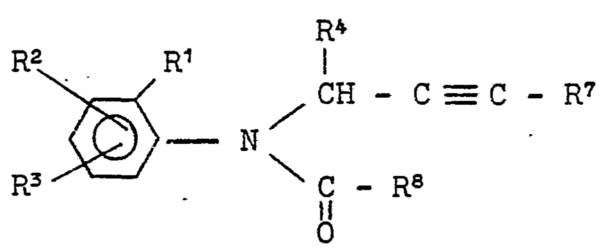

(VIII)

in welcher

$R^1$ bis $R^4$,
$R^7$ und $R^8$ die oben angegebene Bedeutung haben,

mit Wasserstoff in Gegenwart eines Katalysators und gegebenenfalls in Gegenwart eines polaren Lösungsmittels selektiv hydriert, oder

e) N-Propargyl-acetanilide der Formel

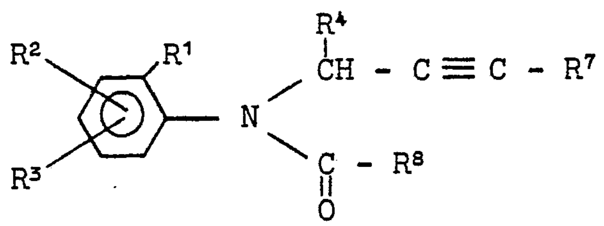

(VIII)

in welcher

$R^1$ bis $R^4$,
$R^7$ und $R^8$ die oben angegebene Bedeutung haben,

mit Halogenen bzw. Hydrohalogeniden der Formel

$$X - Y \qquad (IX)$$

in welcher

X für Wasserstoff oder Halogen steht und
Y für Halogen steht,

in Gegenwart eines Katalysators und gegebenenfalls in Gegenwart eines Lösungsmittels umsetzt, oder

f) Halogenacetanilide der Formel

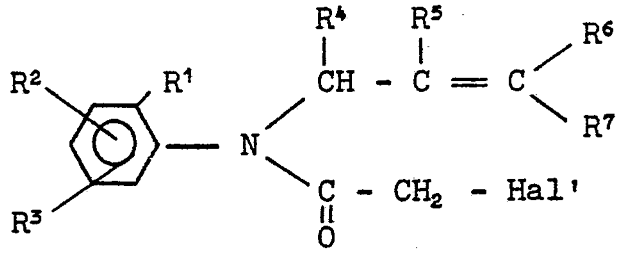

(X)

in welcher

| | |
|---|---|
| $R^1$ bis $R^7$ | die oben angegebene Bedeutung haben und |
| Hal' | für Chlor, Brom oder Jod steht |

mit Verbindungen der Formel

$$B - R^{10} \qquad (XI)$$

in welcher

| | |
|---|---|
| B | für Wasserstoff oder ein Alkalimetall steht und |
| $R^{10}$ | für Az und die Gruppierung $-OR^9$ oder $-SR_9$ steht, |

in Gegenwart eines Verdünnungsmittels und gegebenenfalls in Gegenwart eines Säurebinders umsetzt, oder

g) substituierte N-Allyl-hydroxyacetanilide der Formel

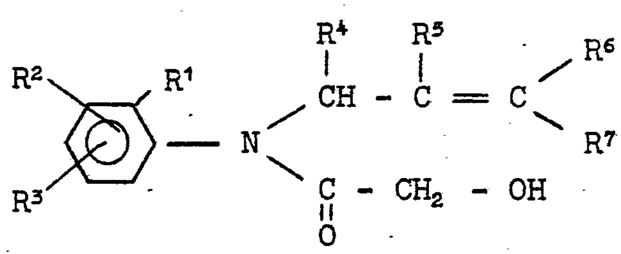

(XII)

in welcher

$R^1$ bis $R^7$ die oben angegebene Bedeutung haben,

(α) gegebenenfalls nach Aktivierung mittels Alkalimetall mit Halogeniden der Formel

$R^{11}$ - Hal' (XIII)

in welcher

Hal' die oben angegebene Bedeutung hat und

$R^{11}$ für den Rest $R^9$ sowie die Gruppe $-SO_2-R^9$ steht, wobei $R^9$ die oben angegebene Bedeutung hat,

in Gegenwart eines organischen Verdünnungsmittels und gegebenenfalls in Gegenwart eines Säurebinders oder in einem wässrig-organischen Zweiphasensystem in Gegenwart eines Phasentransferkatalysators umsetzt, oder

(ß) mit Dihydropyran der Formel

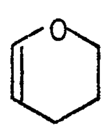

(XIV)

in Gegenwart eines Verdünnungsmittels und gegebenenfalls in Gegenwart eines Katalysators umsetzt, oder

h) Hydroxyacetanilide der Formel

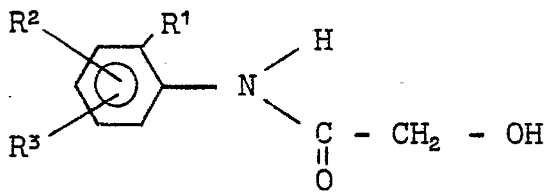

(XV)

in welcher

$R^1$ bis $R^3$ die oben angegebene Bedeutung haben,

gegebenenfalls nach Aktivierung mittels Alkalimetall mit Allylhalogeniden der Formel

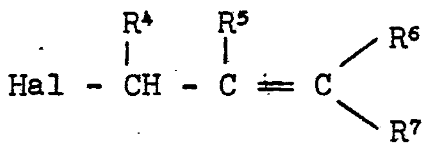

(V)

in welcher

$R^4$ bis $R^7$
und Hal die oben angegebene Bedeutung haben,

gegebenenfalls in Gegenwart eines Säurebinders und in Gegenwart eines organischen Verdünnungsmittels oder in einem wässrig-organischen Zweiphasensystem in Gegenwart eines Phasentransferkatalysators umsetzt.

Überraschenderweise zeigen die neuen substituierten N-Allyl-acetanilide der Formel (I) eine bessere fungizide Wirksamkeit, insbesondere gegen Phytophthora-Arten, als die aus dem Stande der Technik bekannten N-Chloracetyl-N-(2,6-dialkylphenyl)-alanin- bzw. -glycin-alkylester, welche chemisch und wirkungsmäßig naheliegende Verbindungen sind. Die erfindungsgemäßen Stoffe stellen somit eine Bereicherung der Technik dar.

Die erfindungsgemäßen substituierten N-Allyl-acetanilide sind durch die Formel (I) allgemein definiert. In dieser Formel stehen $R^1$, $R^2$ und $R^3$ vorzugsweise für Wasserstoff sowie geradkettiges oder verzweigtes Alkyl, Alkoxy oder Alkylthioalkyl mit jeweils 1 bis 4 Kohlenstoffatomen im Alkylteil. $R^1$ steht außerdem vorzugsweise für Halogen, wie insbesondere Fluor, Chlor oder Brom. $R^4$ steht vorzugsweise für Wasserstoff, geradkettiges oder verzweigtes Alkyl mit 1 bis 4 Kohlenstoffatomen sowie Alkoxyalkyl mit 1 bis 4 Kohlenstoffatomen in jedem Alkylteil. $R^5$ steht vorzugsweise für Wasserstoff, geradkettiges oder verzweigtes Alkyl mit 1 bis 4 Kohlenstoffatomen, Halogen, sowie für gegebenenfalls durch Halogen, Alkyl mit 1 bis 4 Kohlenstoffatomen oder Nitro substituiertes Phenyl. $R^6$ und $R^7$ stehen vorzugsweise für Wasserstoff, geradkettiges oder verzweigtes Alkyl mit 1 bis 4 Kohlenstoffatomen, Halogen, sowie für gegebenenfalls durch Halogen, Alkyl mit 1 bis 4 Kohlenstoffatomen oder Nitro substituiertes Phenyl.

$R^8$ steht vorzugsweise für Furyl, Tetrahydrofuryl, Thiophenyl, Tetrahydro-thio-phenyl, für gegebenenfalls durch Methyl oder Ethyl substituiertes Isoxazolyl sowie für gegebenenfalls durch Cyano oder Thiocyano substituiertes Alkyl mit 1 bis 4 und Alkenyl sowie Alkinyl mit jeweils 2 bis 4 Kohlenstoffatomen; ferner vorzugsweise für Dihalogenalkyl mit 1 bis 2 Kohlenstoffatomen, wobei als Halogenatome vorzugsweise Fluor und Chlor genannt seien, sowie für die Gruppierungen $-CH_2-Az$, $-CH_2-OR^9$, $-CH_2-SR^9$, $-OR^9$, $-SR^9$, $-CH_2-OSO_2R^9$, $-COOR^9$ und $-CH_2-O-$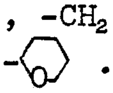 .

Az steht dabei vorzugsweise für Pyrazol-1-yl, 1,2,4-Triazol-1-yl und Imidazol-1-yl. $R^9$ steht vorzugsweise für gegebenenfalls durch Halogen, insbesondere Fluor, Chlor und Brom, Cyano und Thiocyano substituiertes Alkyl mit 1 bis 4 und Alkenyl sowie Alkinyl mit jeweils 2 bis 4 Kohlenstoffatomen in jedem Alkylteil.

Ganz besonders bevorzugt sind diejenigen substituierten N-Allyl-acetanilide der Formel (I), in denen $\underline{R}^1$, $\underline{R}^2$ und $\underline{R}^3$ für Wasserstoff, Methyl, Ethyl, Isopropyl, sek.-Butyl, tert.-Butyl, Methoxymethyl, Ethoxymethyl und Methylthiomethyl stehen; $\underline{R}^1$ außerdem für Chlor oder Brom steht; $\underline{R}^4$ für Wasserstoff, Methyl,Ethyl, Methoxymethyl oder Ethoxymethyl steht; $\underline{R}^5$, $\underline{R}^6$ und $\underline{R}^7$ für Wasserstoff, Methyl, Ethyl, Phenyl, Fluor, Brom, Chlor oder Jod stehen; und $\underline{R}^8$ für 2-Furyl, 2-Thienyl, 2-Tetrahydrofuryl, 5-Methylisoxazol-3-yl, Methoxymethyl, Ethoxymethyl, Allyloxymethyl, Propargyloxymethyl, Ethoxymethoxymethyl, Methylmercaptomethyl, Methoxy, Ethoxy, Methylmercapto, Methylsulfonyloxymethyl, Methoxycarbonyl, Ethoxycarbonyl, Dichlormethyl, Pyrazol-1-yl-methyl, Imidazol-1-yl-methyl, 1,2,4-Triazol-1-yl-methyl und Tetrahydropyran-2-yl-oxy-methyl steht.

Im einzelnen seien außer den bei den Herstellungsbeispielen genannten Verbindungen die folgenden Verbindungen der allgemeinen Formel (I) genannt:

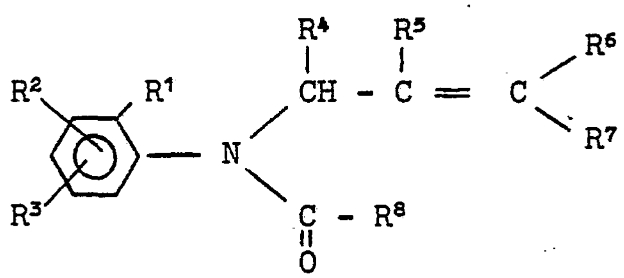

(I)

| $R^1$ | $R^2$ | $R^3$ | $R^4$ | $R^5$ | $R^6$ | $R^7$ | $R^8$ |
|---|---|---|---|---|---|---|---|
| $CH_3$ | $6-CH_3$ | H | H | H | H | H | 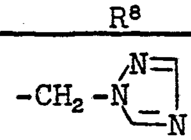 |
| $CH_3$ | $6-CH_3$ | H | H | H | H | H | 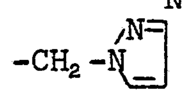 |
| $CH_3$ | $6-CH_3$ | H | H | H | H | H | $-CH_2-O-C_2H_5$ |
| $CH_3$ | $6-CH_3$ | H | H | H | H | H | $-CH_2-O-CH_2-O-C_2H_5$ |
| $CH_3$ | $6-CH_3$ | H | H | H | H | H | 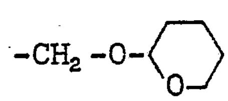 |
| $CH_3$ | $6-CH_3$ | H | H | H | H | H | 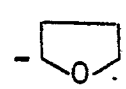 |

| $R^1$ | $R^2$ | $R^3$ | $R^4$ | $R^5$ | $R^6$ | $R^7$ | $R^8$ |
|---|---|---|---|---|---|---|---|
| $CH_3$ | 6-$CH_3$ | H | H | H | H | H | - 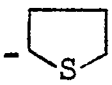 |
| $CH_3$ | 6-$CH_3$ | H | H | H | H | H | $-CH_2-S-CH_3$ |
| $CH_3$ | 6-$CH_3$ | H | H | H | H | H | $-CHCl_2$ |
| $CH_3$ | 6-$CH_3$ | H | H | H | H | H | $-COOCH_3$ |
| $CH_3$ | 6-$CH_3$ | H | H | H | H | H | $-CH_2OSO_2CH_3$ |
| $CH_3$ | 6-$CH_3$ | H | H | $CH_3$ | H | H | $-CH_2-$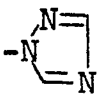 |
| $CH_3$ | 6-$CH_3$ | H | H | $CH_3$ | H | H | $-CH_2-$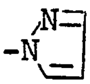 |
| $CH_3$ | 6-$CH_3$ | H | H | $CH_3$ | H | H | $-CH_2-$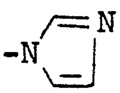 |
| $CH_3$ | 6-$CH_3$ | H | H | $CH_3$ | H | H | $-CH_2OC_2H_5$ |
| $CH_3$ | 6-$CH_3$ | H | H | $CH_3$ | H | H | $-CH_2OCH_2OC_2H_5$ |
| $CH_3$ | 6-$CH_3$ | H | H | $CH_3$ | H | H | $-CH_2O-$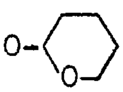 |
| $CH_3$ | 6-$CH_3$ | H | H | $CH_3$ | H | H | - 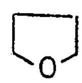 |
| $CH_3$ | 6-$CH_3$ | H | H | $CH_3$ | H | H | - 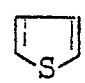 |
| $CH_3$ | 6-$CH_3$ | H | H | $CH_3$ | H | H | - 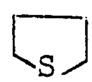 |
| $CH_3$ | 6-$CH_3$ | H | H | $CH_3$ | H | H | $-CH_2SCH_3$ |
| $CH_3$ | 6-$CH_3$ | H | H | $CH_3$ | H | H | $-CH_2SC_2H_5$ |
| $CH_3$ | 6-$CH_3$ | H | H | $CH_3$ | H | H | $-CHCl_2$ |
| $CH_3$ | 6-$CH_3$ | H | H | $CH_3$ | H | H | $-COOCH_3$ |
| $CH_3$ | 6-$CH_3$ | H | H | $CH_3$ | H | H | $-CH_2OSO_2CH_3$ |

| $R^1$ | $R^2$ | $R^3$ | $R^4$ | $R^5$ | $R^6$ | $R^7$ | $R^8$ |
|---|---|---|---|---|---|---|---|
| $CH_3$ | 6-$CH_3$ | H | $CH_3$ | H | H | H | 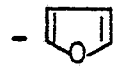 |
| $CH_3$ | 6-$CH_3$ | H | $CH_3$ | H | H | H | $-CH_2OCH_3$ |
| $CH_3$ | 6-$CH_3$ | H | $CH_3$ | H | H | H | $-CH_2-$ 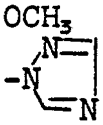 |
| $CH_3$ | 6-$CH_3$ | H | $CH_3$ | H | H | H | $-CH_2-$ 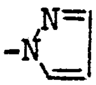 |
| $CH_3$ | 6-$CH_3$ | H | $CH_3$ | H | H | H | $-CH_2-$ 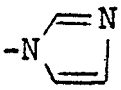 |
| $CH_3$ | 6-$CH_3$ | H | $CH_3$ | H | H | H | $-CH_2OC_2H_5$ |
| $CH_3$ | 6-$CH_3$ | H | $CH_3$ | H | H | H | $-CH_2-O-CH_2-OC_2H_5$ |
| $CH_3$ | 6-$CH_3$ | H | $CH_3$ | H | H | H | $-CH_2O-$ 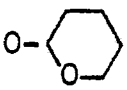 |
| $CH_3$ | 6-$CH_3$ | H | $CH_3$ | H | H | H | 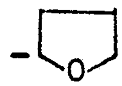 |
| $CH_3$ | 6-$CH_3$ | H | $CH_3$ | H | H | H | 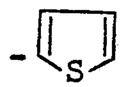 |
| $CH_3$ | 6-$CH_3$ | H | $CH_3$ | H | H | H | 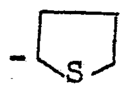 |
| $CH_3$ | 6-$CH_3$ | H | $CH_3$ | H | H | H | $-CH_2SCH_3$ |
| $CH_3$ | 6-$CH_3$ | H | $CH_3$ | H | H | H | $-CH_2SC_2H_5$ |
| $CH_3$ | 6-$CH_3$ | H | $CH_3$ | H | H | H | $-CHCl_2$ |
| $CH_3$ | 6-$CH_3$ | H | $CH_3$ | H | H | H | $-COOCH_3$ |
| $CH_3$ | 6-$CH_3$ | H | $CH_3$ | H | H | H | $-CH_2OSO_2CH_3$ |

| $R^1$ | $R^2$ | $R^3$ | $R^4$ | $R^5$ | $R^6$ | $R^7$ | $R^8$ |
|---|---|---|---|---|---|---|---|
| $CH_3$ | 6-$CH_3$ | H | H | H | $CH_3$ | H | - 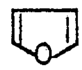 |
| $CH_3$ | 6-$CH_3$ | H | H | H | $CH_3$ | H | $-CH_2OCH_3$ |
| $CH_3$ | 6-$CH_3$ | H | H | H | $CH_3$ | H | $-CH_2-$ 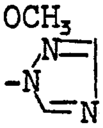 |
| $CH_3$ | 6-$CH_3$ | H | H | H | $CH_3$ | H | $-CH_2-$ 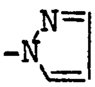 |
| $CH_3$ | 6-$CH_3$ | H | H | H | $CH_3$ | H | $-CH_2-$ 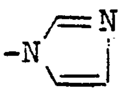 |
| $CH_3$ | 6-$CH_3$ | H | H | H | $CH_3$ | H | $-CH_2OC_2H_5$ |
| $CH_3$ | 6-$CH_3$ | H | H | H | $CH_3$ | H | $-CH_2-O-CH_2-OC_2H_5$ |
| $CH_3$ | 6-$CH_3$ | H | H | H | $CH_3$ | H | $-CH_2O-$ 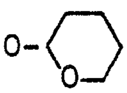 |
| $CH_3$ | 6-$CH_3$ | H | H | H | $CH_3$ | H | - 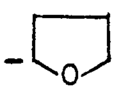 |
| $CH_3$ | 6-$CH_3$ | H | H | H | $CH_3$ | H | - 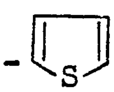 |
| $CH_3$ | 6-$CH_3$ | H | H | H | $CH_3$ | H | - 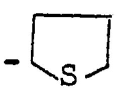 |
| $CH_3$ | 6-$CH_3$ | H | H | H | $CH_3$ | H | $-CH_2SCH_3$ |
| $CH_3$ | 6-$CH_3$ | H | H | H | $CH_3$ | H | $-CH_2SC_2H_5$ |
| $CH_3$ | 6-$CH_3$ | H | H | H | $CH_3$ | H | $-CHCl_2$ |
| $CH_3$ | 6-$CH_3$ | H | H | H | $CH_3$ | H | $-COOCH_3$ |
| $CH_3$ | 6-$CH_3$ | H | H | H | $CH_3$ | H | $-CH_2OSO_2CH_3$ |

| $R^1$ | $R^2$ | $R^3$ | $R^4$ | $R^5$ | $R^6$ | $R^7$ | $R^8$ |
|---|---|---|---|---|---|---|---|
| $CH_3$ | 6-$CH_3$ | H | $CH_3$ | H | $CH_3$ | H | - 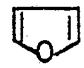 |
| $CH_3$ | 6-$CH_3$ | H | $CH_3$ | H | $CH_3$ | H | $-CH_2OCH_3$ |
| $CH_3$ | 6-$CH_3$ | H | $CH_3$ | H | $CH_3$ | H | $-CH_2-$ 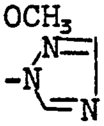 |
| $CH_3$ | 6-$CH_3$ | H | $CH_3$ | H | $CH_3$ | H | $-CH_2-$ 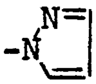 |
| $CH_3$ | 6-$CH_3$ | H | $CH_3$ | H | $CH_3$ | H | $-CH_2-$ 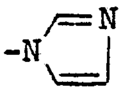 |
| $CH_3$ | 6-$CH_3$ | H | $CH_3$ | H | $CH_3$ | H | $-CH_2OC_2H_5$ |
| $CH_3$ | 6-$CH_3$ | H | $CH_3$ | H | $CH_3$ | H | $-CH_2-O-CH_2-OC_2H_5$ |
| $CH_3$ | 6-$CH_3$ | H | $CH_3$ | H | $CH_3$ | H | $-CH_2O-$ 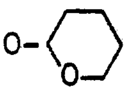 |
| $CH_3$ | 6-$CH_3$ | H | $CH_3$ | H | $CH_3$ | H | - 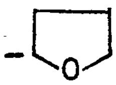 |
| $CH_3$ | 6-$CH_3$ | H | $CH_3$ | H | $CH_3$ | H | - 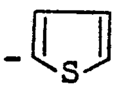 |
| $CH_3$ | 6-$CH_3$ | H | $CH_3$ | H | $CH_3$ | H | - 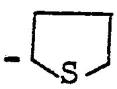 |
| $CH_3$ | 6-$CH_3$ | H | $CH_3$ | H | $CH_3$ | H | $-CH_2SCH_3$ |
| $CH_3$ | 6-$CH_3$ | H | $CH_3$ | H | $CH_3$ | H | $-CH_2SC_2H_5$ |
| $CH_3$ | 6-$CH_3$ | H | $CH_3$ | H | $CH_3$ | H | $-CHCl_2$ |
| $CH_3$ | 6-$CH_3$ | H | $CH_3$ | H | $CH_3$ | H | $-COOCH_3$ |
| $CH_3$ | 6-$CH_3$ | H | $CH_3$ | H | $CH_3$ | H | $-CH_2OSO_2CH_3$ |

| $R^1$ | $R^2$ | $R^3$ | $R^4$ | $R^5$ | $R^6$ | $R^7$ | $R^8$ |
|---|---|---|---|---|---|---|---|
| $CH_3$ | 6-$CH_3$ | H | H | Cl(H) | H(Cl) | H | - 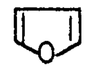 |
| $CH_3$ | 6-$CH_3$ | H | H | Cl(H) | H(Cl) | H | $-CH_2OCH_3$ |
| $CH_3$ | 6-$CH_3$ | H | H | Cl(H) | H(Cl) | H | $-CH_2-$ 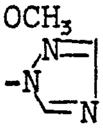 |
| $CH_3$ | 6-$CH_3$ | H | H | Cl(H) | H(Cl) | H | $-CH_2-$ 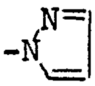 |
| $CH_3$ | 6-$CH_3$ | H | H | Cl(H) | H(Cl) | H | $-CH_2-$ 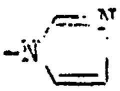 |
| $CH_3$ | 6-$CH_3$ | H | H | Cl(H) | H(Cl) | H | $-CH_2OC_2H_5$ |
| $CH_3$ | 6-$CH_3$ | H | H | Cl(H) | H(Cl) | H | $-CH_2-O-CH_2-OC_2H_5$ |
| $CH_3$ | 6-$CH_3$ | H | H | Cl(H) | H(Cl) | H | $-CH_2O-$ 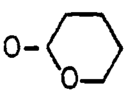 |
| $CH_3$ | 6-$CH_3$ | H | H | Cl(H) | H(Cl) | H | - 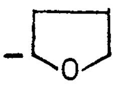 |
| $CH_3$ | 6-$CH_3$ | H | H | Cl(H) | H(Cl) | H | - 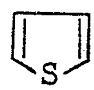 |
| $CH_3$ | 6-$CH_3$ | H | H | Cl(H) | H(Cl) | H | - 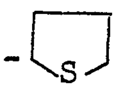 |
| $CH_3$ | 6-$CH_3$ | H | H | Cl(H) | H(Cl) | H | $-CH_2SCH_3$ |
| $CH_3$ | 6-$CH_3$ | H | H | Cl(H) | H(Cl) | H | $-CH_2SC_2H_5$ |
| $CH_3$ | 6-$CH_3$ | H | H | Cl(H) | H(Cl) | H | $-CHCl_2$ |
| $CH_3$ | 6-$CH_3$ | H | H | Cl(H) | H(Cl) | H | $-COOCH_3$ |
| $CH_3$ | 6-$CH_3$ | H | H | Cl(H) | H(Cl) | H | $-CH_2OSO_2CH_3$ |

| $R^1$ | $R^2$ | $R^3$ | $R^4$ | $R^5$ | $R^6$ | $R^7$ | $R^8$ |
|---|---|---|---|---|---|---|---|
| $CH_3$ | 6-$CH_3$ | H | $CH_3$ | Cl(H) | H(Cl) | H | - 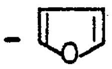 |
| $CH_3$ | 6-$CH_3$ | H | $CH_3$ | Cl(H) | H(Cl) | H | $-CH_2OCH_3$ |
| $CH_3$ | 6-$CH_3$ | H | $CH_3$ | Cl(H) | H(Cl) | H | $-CH_2-$ 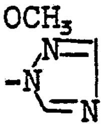 |
| $CH_3$ | 6-$CH_3$ | H | $CH_3$ | Cl(H) | H(Cl) | H | $-CH_2-$ 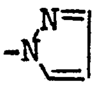 |
| $CH_3$ | 6-$CH_3$ | H | $CH_3$ | Cl(H) | H(Cl) | H | $-CH_2-$ 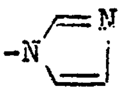 |
| $CH_3$ | 6-$CH_3$ | H | $CH_3$ | Cl(H) | H(Cl) | H | $-CH_2OC_2H_5$ |
| $CH_3$ | 6-$CH_3$ | H | $CH_3$ | Cl(H) | H(Cl) | H | $-CH_2-O-CH_2-OC_2H_5$ |
| $CH_3$ | 6-$CH_3$ | H | $CH_3$ | Cl(H) | H(Cl) | H | $-CH_2O-$ 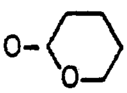 |
| $CH_3$ | 6-$CH_3$ | H | $CH_3$ | Cl(H) | H(Cl) | H | - 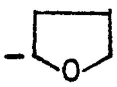 |
| $CH_3$ | 6-$CH_3$ | H | $CH_3$ | Cl(H) | H(Cl) | H | - 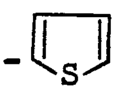 |
| $CH_3$ | 6-$CH_3$ | H | $CH_3$ | Cl(H) | H(Cl) | H | - 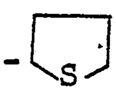 |
| $CH_3$ | 6-$CH_3$ | H | $CH_3$ | Cl(H) | H(Cl) | H | $-CH_2SCH_3$ |
| $CH_3$ | 6-$CH_3$ | H | $CH_3$ | Cl(H) | H(Cl) | H | $-CH_2SC_2H_5$ |
| $CH_3$ | 6-$CH_3$ | H | $CH_3$ | Cl(H) | H(Cl) | H | $-CHCl_2$ |
| $CH_3$ | 6-$CH_3$ | H | $CH_3$ | Cl(H) | H(Cl) | H | $-COOCH_3$ |
| $CH_3$ | 6-$CH_3$ | H | $CH_3$ | Cl(H) | H(Cl) | H | $-CH_2OSO_2CH_3$ |

| $R^1$ | $R^2$ | $R^3$ | $R^4$ | $R^5$ | $R^6$ | $R^7$ | $R^8$ |
|---|---|---|---|---|---|---|---|
| $CH_3$ | 6-$CH_3$ | H | H | H | Cl | Cl | - 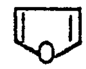 |
| $CH_3$ | 6-$CH_3$ | H | H | H | Cl | Cl | $-CH_2-$ 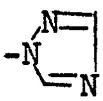 |
| $CH_3$ | 6-$CH_3$ | H | H | H | Cl | Cl | $-CH_2-$ 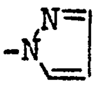 |
| $CH_3$ | 6-$CH_3$ | H | H | H | Cl | Cl | $-CH_2-$ 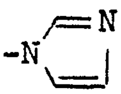 |
| $CH_3$ | 6-$CH_3$ | H | H | H | Cl | Cl | $-CH_2OC_2H_5$ |
| $CH_3$ | 6-$CH_3$ | H | H | H | Cl | Cl | $-CH_2-O-CH_2-OC_2H_5$ |
| $CH_3$ | 6-$CH_3$ | H | H | H | Cl | Cl | $-CH_2O-$ 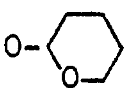 |
| $CH_3$ | 6-$CH_3$ | H | H | H | Cl | Cl | - 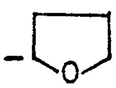 |
| $CH_3$ | 6-$CH_3$ | H | H | H | Cl | Cl | - 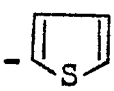 |
| $CH_3$ | 6-$CH_3$ | H | H | H | Cl | Cl | - 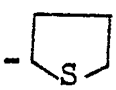 |
| $CH_3$ | 6-$CH_3$ | H | H | H | Cl | Cl | $-CH_2SCH_3$ |
| $CH_3$ | 6-$CH_3$ | H | H | H | Cl | Cl | $-CH_2SC_2H_5$ |
| $CH_3$ | 6-$CH_3$ | H | H | H | Cl | Cl | $-CHCl_2$ |
| $CH_3$ | 6-$CH_3$ | H | H | H | Cl | Cl | $-COOCH_3$ |
| $CH_3$ | 6-$CH_3$ | H | H | H | Cl | Cl | $-CH_2OSO_2CH_3$ |

| $R^1$ | $R^2$ | $R^3$ | $R^4$ | $R^5$ | $R^6$ | $R^7$ | $R^8$ |
|---|---|---|---|---|---|---|---|
| $CH_3$ | 6-$CH_3$ | H | $CH_3$ | H | Cl | Cl | - 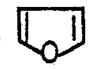 |
| $CH_3$ | 6-$CH_3$ | H | $CH_3$ | H | Cl | Cl | $-CH_2-$ 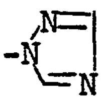 |
| $CH_3$ | 6-$CH_3$ | H | $CH_3$ | H | Cl | Cl | $-CH_2-$ 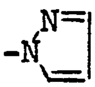 |
| $CH_3$ | 6-$CH_3$ | H | $CH_3$ | H | Cl | Cl | $-CH_2-$ 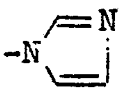 |
| $CH_3$ | 6-$CH_3$ | H | $CH_3$ | H | Cl | Cl | $-CH_2OC_2H_5$ |
| $CH_3$ | 6-$CH_3$ | H | $CH_3$ | H | Cl | Cl | $-CH_2-O-CH_2-OC_2H_5$ |
| $CH_3$ | 6-$CH_3$ | H | $CH_3$ | H | Cl | Cl | $-CH_2O-$ 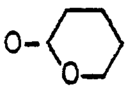 |
| $CH_3$ | 6-$CH_3$ | H | $CH_3$ | H | Cl | Cl | - 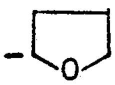 |
| $CH_3$ | 6-$CH_3$ | H | $CH_3$ | H | Cl | Cl | - 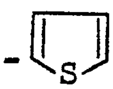 |
| $CH_3$ | 6-$CH_3$ | H | $CH_3$ | H | Cl | Cl | - 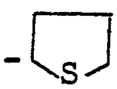 |
| $CH_3$ | 6-$CH_3$ | H | $CH_3$ | H | Cl | Cl | $-CH_2SCH_3$ |
| $CH_3$ | 6-$CH_3$ | H | $CH_3$ | H | Cl | Cl | $-CH_2SC_2H_5$ |
| $CH_3$ | 6-$CH_3$ | H | $CH_3$ | H | Cl | Cl | $-CHCl_2$ |
| $CH_3$ | 6-$CH_3$ | H | $CH_3$ | H | Cl | Cl | $-COOCH_3$ |
| $CH_3$ | 6-$CH_3$ | H | $CH_3$ | H | Cl | Cl | $-CH_2OSO_2CH_3$ |

| $R^1$ | $R^2$ | $R^3$ | $R^4$ | $R^5$ | $R^6$ | $R^7$ | $R^8$ |
|---|---|---|---|---|---|---|---|
| $CH_3$ | 6-$CH_3$ | H | H | H | Cl | $CH_3$ | - 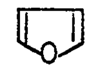 |
| $CH_3$ | 6-$CH_3$ | H | H | H | Cl | $CH_3$ | $-CH_2-$ 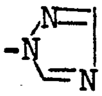 |
| $CH_3$ | 6-$CH_3$ | H | H | H | Cl | $CH_3$ | $-CH_2-$ 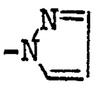 |
| $CH_3$ | 6-$CH_3$ | H | H | H | Cl | $CH_3$ | $-CH_2-$ 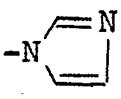 |
| $CH_3$ | 6-$CH_3$ | H | H | H | Cl | $CH_3$ | $-CH_2OC_2H_5$ |
| $CH_3$ | 6-$CH_3$ | H | H | H | Cl | $CH_3$ | $-CH_2-O-CH_2-OC_2H_5$ |
| $CH_3$ | 6-$CH_3$ | H | H | H | Cl | $CH_3$ | $-CH_2O-$ 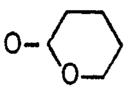 |
| $CH_3$ | 6-$CH_3$ | H | H | H | Cl | $CH_3$ | - 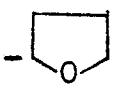 |
| $CH_3$ | 6-$CH_3$ | H | H | H | Cl | $CH_3$ | - 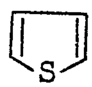 |
| $CH_3$ | 6-$CH_3$ | H | H | H | Cl | $CH_3$ | - 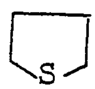 |
| $CH_3$ | 6-$CH_3$ | H | H | H | Cl | $CH_3$ | $-CH_2SCH_3$ |
| $CH_3$ | 6-$CH_3$ | H | H | H | Cl | $CH_3$ | $-CH_2SC_2H_5$ |
| $CH_3$ | 6-$CH_3$ | H | H | H | Cl | $CH_3$ | $-CHCl_2$ |
| $CH_3$ | 6-$CH_3$ | H | H | H | Cl | $CH_3$ | $-COOCH_3$ |
| $CH_3$ | 6-$CH_3$ | H | H | H | Cl | $CH_3$ | $-CH_2OSO_2CH_3$ |

| $R^1$ | $R^2$ | $R^3$ | $R^4$ | $R^5$ | $R^6$ | $R^7$ | $R^8$ |
|---|---|---|---|---|---|---|---|
| $CH_3$ | 6-$CH_3$ | H | $CH_3$ | H | Cl | $CH_3$ | - 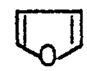 |
| $CH_3$ | 6-$CH_3$ | H | $CH_3$ | H | Cl | $CH_3$ | $-CH_2-$ 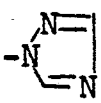 |
| $CH_3$ | 6-$CH_3$ | H | $CH_3$ | H | Cl | $CH_3$ | $-CH_2-$ 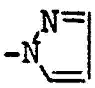 |
| $CH_3$ | 6-$CH_3$ | H | $CH_3$ | H | Cl | $CH_3$ | $-CH_2-$ 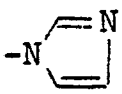 |
| $CH_3$ | 6-$CH_3$ | H | $CH_3$ | H | Cl | $CH_3$ | $-CH_2OC_2H_5$ |
| $CH_3$ | 6-$CH_3$ | H | $CH_3$ | H | Cl | $CH_3$ | $-CH_2-O-CH_2-OC_2H_5$ |
| $CH_3$ | 6-$CH_3$ | H | $CH_3$ | H | Cl | $CH_3$ | $-CH_2O-$ 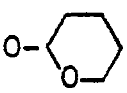 |
| $CH_3$ | 6-$CH_3$ | H | $CH_3$ | H | Cl | $CH_3$ | - 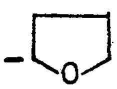 |
| $CH_3$ | 6-$CH_3$ | H | $CH_3$ | H | Cl | $CH_3$ | - 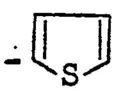 |
| $CH_3$ | 6-$CH_3$ | H | $CH_3$ | H | Cl | $CH_3$ | - 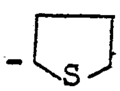 |
| $CH_3$ | 6-$CH_3$ | H | $CH_3$ | H | Cl | $CH_3$ | $-CH_2SCH_3$ |
| $CH_3$ | 6-$CH_3$ | H | $CH_3$ | H | Cl | $CH_3$ | $-CH_2SC_2H_5$ |
| $CH_3$ | 6-$CH_3$ | H | $CH_3$ | H | Cl | $CH_3$ | $-CHCl_2$ |
| $CH_3$ | 6-$CH_3$ | H | $CH_3$ | H | Cl | $CH_3$ | $-COOCH_3$ |
| $CH_3$ | 6-$CH_3$ | H | $CH_3$ | H | Cl | $CH_3$ | $-CH_2OSO_2CH_3$ |

| $R^1$ | $R^2$ | $R^3$ | $R^4$ | $R^5$ | $R^6$ | $R^7$ | $R^8$ |
|---|---|---|---|---|---|---|---|
| $CH_3$ | $6-C_2H_5$ | H | H | H | H | H | 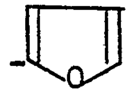 |
| $CH_3$ | $6-C_2H_5$ | H | H | H | H | H | $-CH_2OCH_3$ |
| $CH_3$ | $6-C_2H_5$ | H | H | H | H | H | $-CH_2-$ 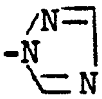 |
| $CH_3$ | $6-C_2H_5$ | H | H | H | H | H | $-CH_2-$ 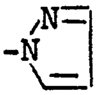 |
| $CH_3$ | $6-C_2H_5$ | H | H | H | H | H | $-CH_2OC_2H_5$ |
| $CH_3$ | $6-C_2H_5$ | H | H | H | H | H | $-CH_2OSO_2CH_3$ |
| $CH_3$ | $6-C_2H_5$ | H | H | H | H | H | $-CHCl_2$ |
| $CH_3$ | $6-C_2H_5$ | H | H | H | H | H | $-COOCH_3$ |
| $CH_3$ | $6-C_2H_5$ | H | H | H | H | H | 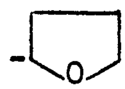 |
| $CH_3$ | $6-C_2H_5$ | H | H | H | H | H | $-CH_2SCH_3$ |
| $CH_3$ | $6-C_2H_5$ | H | H | H | H | H | $-CH_2-O-$ 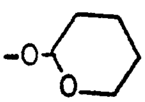 |

| $R^1$ | $R^2$ | $R^3$ | $R^4$ | $R^5$ | $R^6$ | $R^7$ | $R^8$ |
|---|---|---|---|---|---|---|---|
| $CH_3$ | $6-C_2H_5$ | H | $CH_3$ | H | H | H | 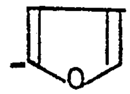 |
| $CH_3$ | $6-C_2H_5$ | H | $CH_3$ | H | H | H | $-CH_2OCH_3$ |
| $CH_3$ | $6-C_2H_5$ | H | $CH_3$ | H | H | H | $-CH_2-$ 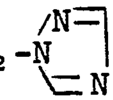 |
| $CH_3$ | $6-C_2H_5$ | H | $CH_3$ | H | H | H | $-CH_2-$ 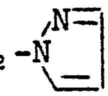 |
| $CH_3$ | $6-C_2H_5$ | H | $CH_3$ | H | H | H | $-CH_2OC_2H_5$ |
| $CH_3$ | $6-C_2H_5$ | H | $CH_3$ | H | H | H | $-CH_2OSO_2CH_3$ |
| $CH_3$ | $6-C_2H_5$ | H | $CH_3$ | H | H | H | $-CHCl_2$ |
| $CH_3$ | $6-C_2H_5$ | H | $CH_3$ | H | H | H | $-COOCH_3$ |
| $CH_3$ | $6-C_2H_5$ | H | $CH_3$ | H | H | H | 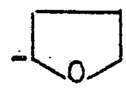 |
| $CH_3$ | $6-C_2H_5$ | H | $CH_3$ | H | H | H | $-CH_2SCH_3$ |
| $CH_3$ | $6-C_2H_5$ | H | $CH_3$ | H | H | H | $-CH_2-O-$ 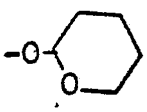 |

| $R^1$ | $R^2$ | $R^3$ | $R^4$ | $R^5$ | $R^6$ | $R^7$ | $R^8$ |
|---|---|---|---|---|---|---|---|
| $CH_3$ | $6-C_2H_5$ | H | H | $CH_3$ | H | H | 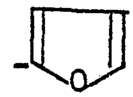 |
| $CH_3$ | $6-C_2H_5$ | H | H | $CH_3$ | H | H | $-CH_2OCH_3$ |
| $CH_3$ | $6-C_2H_5$ | H | H | $CH_3$ | H | H | $-CH_2-$ 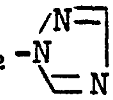 |
| $CH_3$ | $6-C_2H_5$ | H | H | $CH_3$ | H | H | $-CH_2-$ 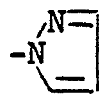 |
| $CH_3$ | $6-C_2H_5$ | H | H | $CH_3$ | H | H | $-CH_2OC_2H_5$ |
| $CH_3$ | $6-C_2H_5$ | H | H | $CH_3$ | H | H | $-CH_2OSO_2CH_3$ |
| $CH_3$ | $6-C_2H_5$ | H | H | $CH_3$ | H | H | $-CHCl_2$ |
| $CH_3$ | $6-C_2H_5$ | H | H | $CH_3$ | H | H | $-COOCH_3$ |
| $CH_3$ | $6-C_2H_5$ | H | H | $CH_3$ | H | H | 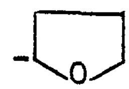 |
| $CH_3$ | $6-C_2H_5$ | H | H | $CH_3$ | H | H | $-CH_2SCH_3$ |
| $CH_3$ | $6-C_2H_5$ | H | H | $CH_3$ | H | H | $-CH_2-O-$ 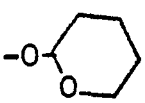 |

| $R^1$ | $R^2$ | $R^3$ | $R^4$ | $R^5$ | $R^6$ | $R^7$ | $R^8$ |
|---|---|---|---|---|---|---|---|
| $CH_3$ | $6-C_2H_5$ | H | H | H | Cl | H | 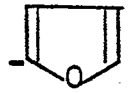 |
| $CH_3$ | $6-C_2H_5$ | H | H | H | Cl | H | $-CH_2OCH_3$ |
| $CH_3$ | $6-C_2H_5$ | H | H | H | Cl | H | $-CH_2-$ 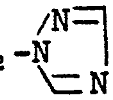 |
| $CH_3$ | $6-C_2H_5$ | H | H | H | Cl | H | $-CH_2-$ 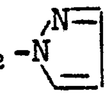 |
| $CH_3$ | $6-C_2H_5$ | H | H | H | Cl | H | $-CH_2OC_2H_5$ |
| $CH_3$ | $6-C_2H_5$ | H | H | H | Cl | H | $-CH_2OSO_2CH_3$ |
| $CH_3$ | $6-C_2H_5$ | H | H | H | Cl | H | $-CHCl_2$ |
| $CH_3$ | $6-C_2H_5$ | H | H | H | Cl | H | $-COOCH_3$ |
| $CH_3$ | $6-C_2H_5$ | H | H | H | Cl | H | 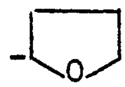 |
| $CH_3$ | $6-C_2H_5$ | H | H | H | Cl | H | $-CH_2SCH_3$ |
| $CH_3$ | $6-C_2H_5$ | H | H | H | Cl | H | $-CH_2-O-$ 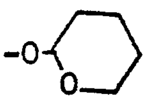 |

| $R^1$ | $R^2$ | $R^3$ | $R^4$ | $R^5$ | $R^6$ | $R^7$ | $R^8$ |
|---|---|---|---|---|---|---|---|
| $C_2H_5$ | $6-C_2H_5$ | H | H | H | H | H | 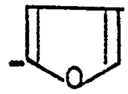 |
| $C_2H_5$ | $6-C_2H_5$ | H | H | H | H | H | $-CH_2OCH_3$ |
| $C_2H_5$ | $6-C_2H_5$ | H | H | H | H | H | $-CH_2-$ 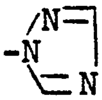 |
| $C_2H_5$ | $6-C_2H_5$ | H | H | H | H | H | $-CH_2-$ 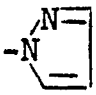 |
| $C_2H_5$ | $6-C_2H_5$ | H | H | H | H | H | $-CH_2OC_2H_5$ |
| $C_2H_5$ | $6-C_2H_5$ | H | H | H | H | H | $-CH_2OSO_2CH_3$ |
| $C_2H_5$ | $6-C_2H_5$ | H | H | H | H | H | $-CHCl_2$ |
| $C_2H_5$ | $6-C_2H_5$ | H | H | H | H | H | $-COOCH_3$ |
| $C_2H_5$ | $6-C_2H_5$ | H | H | H | H | H | 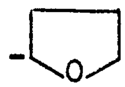 |
| $C_2H_5$ | $6-C_2H_5$ | H | H | H | H | H | $-CH_2SCH_3$ |
| $C_2H_5$ | $6-C_2H_5$ | H | H | H | H | H | $-CH_2-O-$ 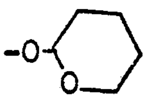 |

| $R^1$ | $R^2$ | $R^3$ | $R^4$ | $R^5$ | $R^6$ | $R^7$ | $R^8$ |
|---|---|---|---|---|---|---|---|
| $C_2H_5$ | $6-C_2H_5$ | H | $CH_3$ | H | H | H | - 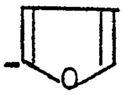 |
| $C_2H_5$ | $6-C_2H_5$ | H | $CH_3$ | H | H | H | $-CH_2OCH_3$ |
| $C_2H_5$ | $6-C_2H_5$ | H | $CH_3$ | H | H | H | $-CH_2-$ 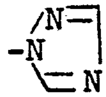 |
| $C_2H_5$ | $6-C_2H_5$ | H | $CH_3$ | H | H | H | $-CH_2-$ 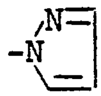 |
| $C_2H_5$ | $6-C_2H_5$ | H | $CH_3$ | H | H | H | $-CH_2OC_2H_5$ |
| $C_2H_5$ | $6-C_2H_5$ | H | $CH_3$ | H | H | H | $-CH_2OSO_2CH_3$ |
| $C_2H_5$ | $6-C_2H_5$ | H | $CH_3$ | H | H | H | $-CHCl_2$ |
| $C_2H_5$ | $6-C_2H_5$ | H | $CH_3$ | H | H | H | $-COOCH_3$ |
| $C_2H_5$ | $6-C_2H_5$ | H | $CH_3$ | H | H | H | - 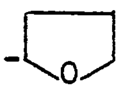 |
| $C_2H_5$ | $6-C_2H_5$ | H | $CH_3$ | H | H | H | $-CH_2SCH_3$ |
| $C_2H_5$ | $6-C_2H_5$ | H | $CH_3$ | H | H | H | $-CH_2-O-$ 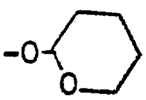 |

| $R^1$ | $R^2$ | $R^3$ | $R^4$ | $R^5$ | $R^6$ | $R^7$ | $R^8$ |
|---|---|---|---|---|---|---|---|
| $C_2H_5$ | $6-C_2H_5$ | H | H | $CH_3$ | H | H | 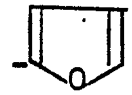 |
| $C_2H_5$ | $6-C_2H_5$ | H | H | $CH_3$ | H | H | $-CH_2OCH_3$ |
| $C_2H_5$ | $6-C_2H_5$ | H | H | $CH_3$ | H | H | $-CH_2-$ 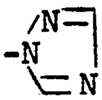 |
| $C_2H_5$ | $6-C_2H_5$ | H | H | $CH_3$ | H | H | $-CH_2-$ 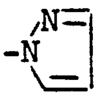 |
| $C_2H_5$ | $6-C_2H_5$ | H | H | $CH_3$ | H | H | $-CH_2OC_2H_5$ |
| $C_2H_5$ | $6-C_2H_5$ | H | H | $CH_3$ | H | H | $-CH_2OSO_2CH_3$ |
| $C_2H_5$ | $6-C_2H_5$ | H | H | $CH_3$ | H | H | $-CHCl_2$ |
| $C_2H_5$ | $6-C_2H_5$ | H | H | $CH_3$ | H | H | $-COOCH_3$ |
| $C_2H_5$ | $6-C_2H_5$ | H | H | $CH_3$ | H | H | 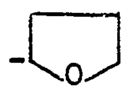 |
| $C_2H_5$ | $6-C_2H_5$ | H | H | $CH_3$ | H | H | $-CH_2SCH_3$ |
| $C_2H_5$ | $6-C_2H_5$ | H | H | $CH_3$ | H | H | $-CH_2-$ 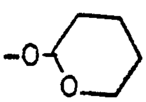 |

| $R^1$ | $R^2$ | $R^3$ | $R^4$ | $R^5$ | $R^6$ | $R^7$ | $R^8$ |
|---|---|---|---|---|---|---|---|
| $C_2H_5$ | $6-C_2H_5$ | H | H | H | Cl | H | 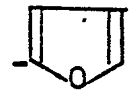 |
| $C_2H_5$ | $6-C_2H_5$ | H | H | H | Cl | H | $-CH_2OCH_3$ |
| $C_2H_5$ | $6-C_2H_5$ | H | H | H | Cl | H | $-CH_2$ - 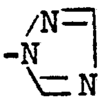 |
| $C_2H_5$ | $6-C_2H_5$ | H | H | H | Cl | H | $-CH_2$ - 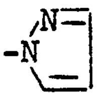 |
| $C_2H_5$ | $6-C_2H_5$ | H | H | H | Cl | H | $-CH_2OC_2H_5$ |
| $C_2H_5$ | $6-C_2H_5$ | H | H | H | Cl | H | $-CH_2OSO_2CH_3$ |
| $C_2H_5$ | $6-C_2H_5$ | H | H | H | Cl | H | $-CHCl_2$ |
| $C_2H_5$ | $6-C_2H_5$ | H | H | H | Cl | H | $-COOCH_3$ |
| $C_2H_5$ | $6-C_2H_5$ | H | H | H | Cl | H | 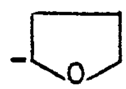 |
| $C_2H_5$ | $6-C_2H_5$ | H | H | H | Cl | H | $-CH_2SCH_3$ |
| $C_2H_5$ | $6-C_2H_5$ | H | H | H | Cl | H | $-CH_2$ -O- 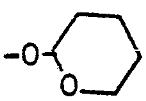 |

| $R^1$ | $R^2$ | $R^3$ | $R^4$ | $R^5$ | $R^6$ | $R^7$ | $R^8$ |
|---|---|---|---|---|---|---|---|
| Cl | 6-$CH_3$ | H | H | H | H | H | 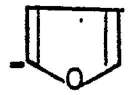 |
| Cl | 6-$CH_3$ | H | H | H | H | H | $-CH_2OCH_3$ |
| Cl | 6-$CH_3$ | H | H | H | H | H | $-CH_2-$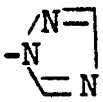 |
| Cl | 6-$CH_3$ | H | H | H | H | H | $-CH_2-$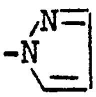 |
| Cl | 6-$CH_3$ | H | H | H | H | H | $-CH_2OC_2H_5$ |
| Cl | 6-$CH_3$ | H | H | H | H | H | $-CH_2OSO_2CH_3$ |
| Cl | 6-$CH_3$ | H | H | H | H | H | $-CHCl_2$ |
| Cl | 6-$CH_3$ | H | H | H | H | H | $-COOCH_3$ |
| Cl | 6-$CH_3$ | H | H | H | H | H | 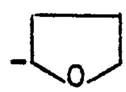 |
| Cl | 6-$CH_3$ | H | H | H | H | H | $-CH_2SCH_3$ |
| Cl | 6-$CH_3$ | H | H | H | H | H | $-CH_2-O-$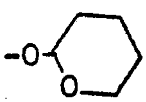 |

| $R^1$ | $R^2$ | $R^3$ | $R^4$ | $R^5$ | $R^6$ | $R^7$ | $R^8$ |
|---|---|---|---|---|---|---|---|
| Cl | 6-$CH_3$ | H | $CH_3$ | H | H | H | 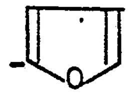 |
| Cl | 6-$CH_3$ | H | $CH_3$ | H | H | H | $-CH_2OCH_3$ |
| Cl | 6-$CH_3$ | H | $CH_3$ | H | H | H | $-CH_2-$ 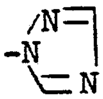 |
| Cl | 6-$CH_3$ | H | $CH_3$ | H | H | H | $-CH_2-$ 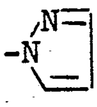 |
| Cl | 6-$CH_3$ | H | $CH_3$ | H | H | H | $-CH_2OC_2H_5$ |
| Cl | 6-$CH_3$ | H | $CH_3$ | H | H | H | $-CH_2OSO_2CH_3$ |
| Cl | 6-$CH_3$ | H | $CH_3$ | H | H | H | $-CHCl_2$ |
| Cl | 6-$CH_3$ | H | $CH_3$ | H | H | H | $-COOCH_3$ |
| Cl | 6-$CH_3$ | H | $CH_3$ | H | H | H | 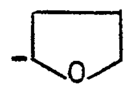 |
| Cl | 6-$CH_3$ | H | $CH_3$ | H | H | H | $-CH_2SCH_3$ |
| Cl | 6-$CH_3$ | H | $CH_3$ | H | H | H | $-CH_2-O-$ 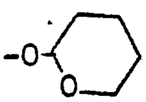 |

Verwendet man beispielsweise 2,6-Dimethyl-N-allyl-anilin und Furan-2-carbonsäurechlorid als Ausgangsstoffe, so kann der Reaktionsablauf durch das folgende Formelschema wiedergegeben werden (Verfahren a):

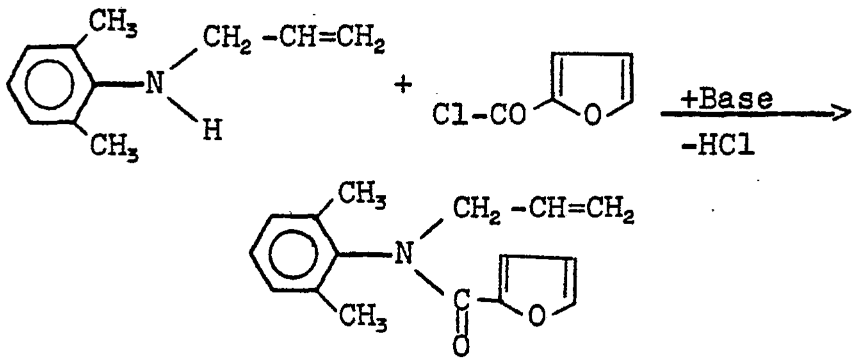


Verwendet man beispielsweise 2,6-Dimethyl-methoxyacetanilid und Allylbromid als Ausgangsstoffe, so kann der Reaktionsablauf durch das folgende Formelschema wiedergegeben werden (Verfahren b):

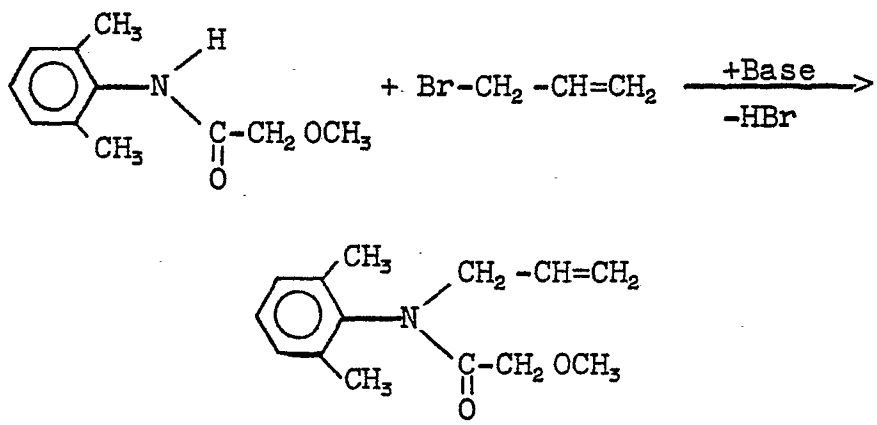


Verwendet man beispielsweise 2,6-Dimethyl-N-(2-furoyl)-N-acetylmethyl-anilin und Triphenyl-phosphin-methylen als Ausgangsstoffe, so kann der Reaktionsablauf durch das folgende Formelschema wiedergegeben werden (Verfahren c):

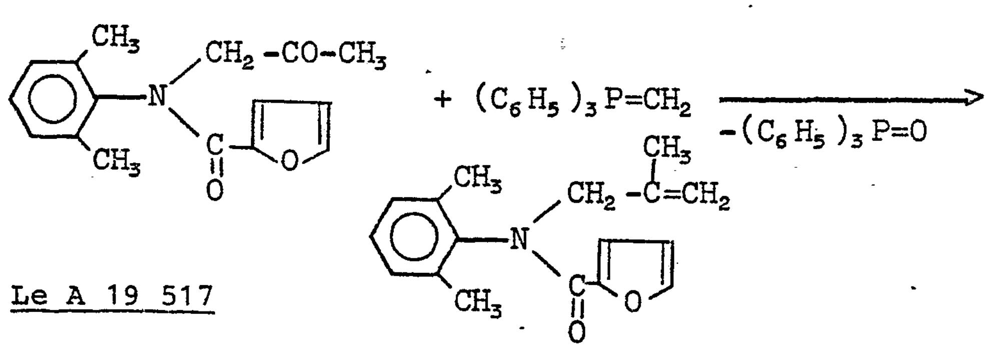

Verwendet man beispielsweise 2,6-Dimethyl-N-propargyl-methoxyacetanilid und Wasserstoff als Ausgangsstoffe, so kann der Reaktionsablauf durch das folgende Formelschema wiedergegeben werden (Verfahren d):

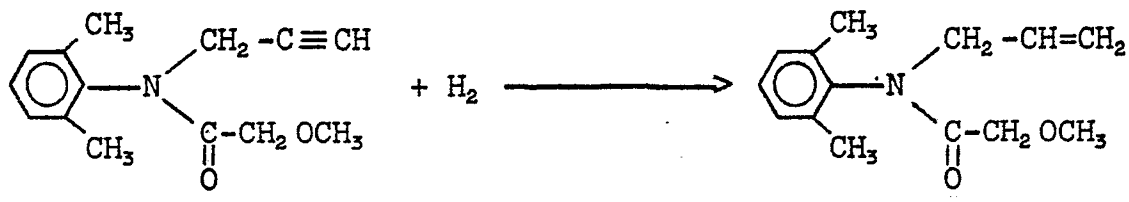

Verwendet man beispielsweise 2,6-Dimethyl-N-propargyl-methoxyacetanilid und Bromwasserstoff als Ausgangsstoffe, so kann der Reaktionsablauf durch das folgende Formelschema wiedergegeben werden(Verfahren e):

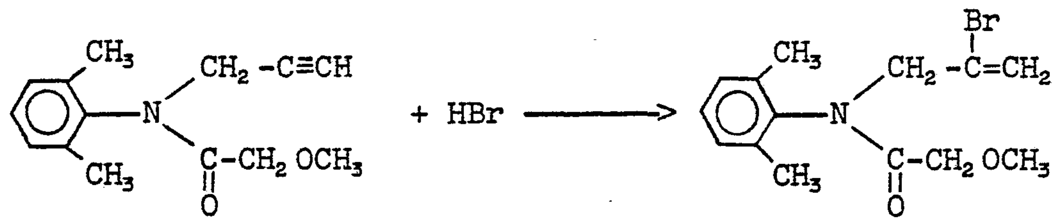

Verwendet man beispielsweise 2,6-Dimethyl-N-allyl-chloracetanilid und Pyrazol als Ausgangsstoffe, so kann der Reaktionsablauf durch das folgende Formelschema wiedergegeben werden (Verfahren f):

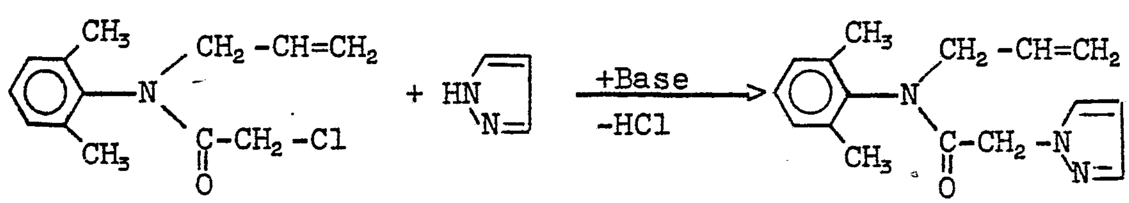


Verwendet man beispielsweise 2,6-Dimethyl-N-allyl-hydroxyacetanilid und Ethoxymethylchlorid als Ausgangsstoffe, so kann der Reaktionsablauf durch das folgende Formelschema wiedergegeben werden (Verfahren g/α):

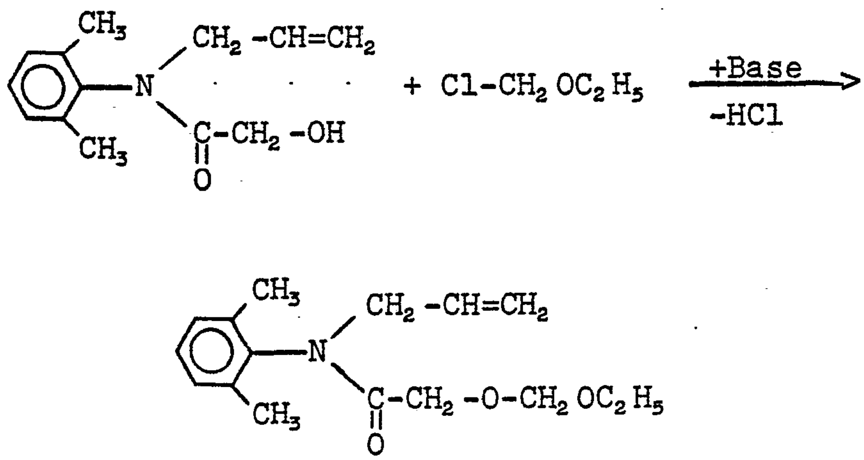


Verwendet man beispielsweise 2,6-Dimethyl-N-allyl-hydroxyacetanilid und 3,4-Dihydro-2H-pyran als Ausgangsstoffe, so kann der Reaktionsablauf durch das folgende Formelschema wiedergegeben werden (Verfahren g/ß):

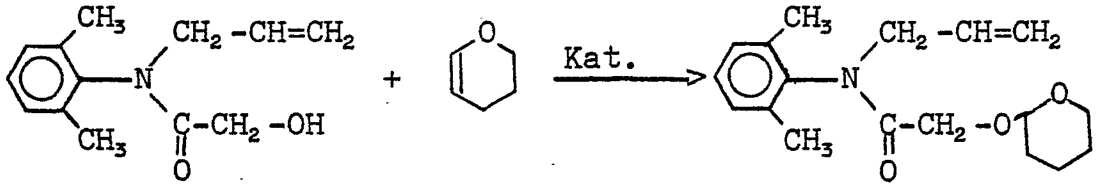


Verwendet man beispielsweise 1 Mol 2,6-Dimethyl-hydroxyacetanilid und 2 Mol Allylbromid als Ausgangsstoffe, so kann der Reaktionsablauf durch das folgende Formelschema wiedergegeben werden (Verfahren h):

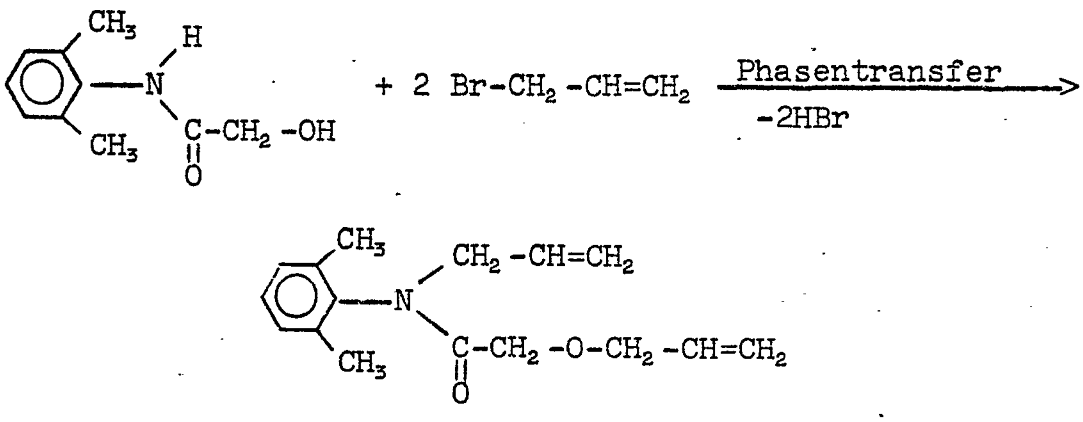

Die bei der Durchführung des erfindungsgemäßen Verfahrens (a) als Ausgangsstoffe zu verwendenden N-Allyl-aniline sind durch die Formel (II) allgemein definiert. In dieser Formel stehen $\underline{R^1}$ $\underline{\text{bis}}$ $\underline{R^7}$ vorzugsweise für diejenigen Reste, die bereits im Zusammenhang mit der Beschreibung der erfindungsgemäßen Stoffe der Formel (I) vorzugsweise für diese Substituenten genannt wurden.

Die N-Allyl-aniline der Formel (II) sind weitgehend bekannt (vergleiche u.a. US-Patentschrift 3 475 156 sowie DE-OS 22 18 097 und DE-OS 28 05 525), bzw. können sie nach bekannten Verfahren erhalten werden, indem man z.B. entsprechende Aniline mit Allylhalogeniden der Formel (V) oder den entsprechenden Allylsulfonaten, wie beispielsweise Mesylaten oder Tosylaten, in Gegenwart eines Säurebinders, wie z.B. Kaliumcarbonat, gegebenenfalls in Gegenwart eines inerten organischen Lösungsmittels, wie beispielsweise Ethanol, bei Temperaturen zwischen 20 und 120°C umsetzt, wobei vorzugsweise auch ein Ueberschuß an Anilin eingesetzt werden kann. Als Beispiele für die Ausgangsstoffe der Formel (II) seien genannt:

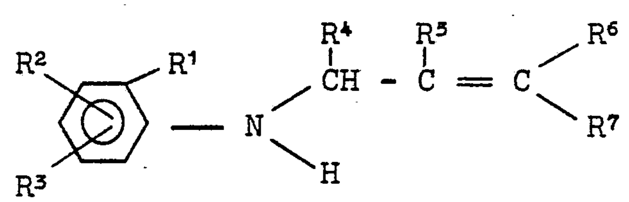

(II)

| $R^1$ | $R^2$ | $R^3$ | $R^4$ | $R^5$ | $R^6$ | $R^7$ |
|---|---|---|---|---|---|---|
| $CH_3$ | 6-$CH_3$ | H | H | H | H | H |
| $C_2H_5$ | 6-$C_2H_5$ | H | H | H | H | H |
| $C_2H_5$ | 6-$CH_3$ | H | H | H | H | H |
| $(CH_3)_3C$ | H | H | H | H | H | H |
| $CH_3$ | 3-$CH_3$ | H | H | H | H | H |
| $CH_3$ | 5-$CH_3$ | H | H | H | H | H |
| Cl | 6-$CH_3$ | H | H | H | H | H |
| Cl | 6-$C(CH_3)_3$ | H | H | H | H | H |
| $CH_3$ | 3-$CH_3$ | 6-$CH_3$ | H | H | H | H |
| $CH_3$ | 6-$CH_3$ | H | $CH_3$ | H | H | H |
| $C_2H_5$ | 6-$C_2H_5$ | H | $CH_3$ | H | H | H |
| $C_2H_5$ | 6-$CH_3$ | H | $CH_3$ | H | H | H |
| Cl | 6-$CH_3$ | H | $CH_3$ | H | H | H |
| $CH_3$ | 6-$CH_3$ | H | H | $CH_3$ | H | H |
| $C_2H_5$ | 6-$C_2H_5$ | H | H | $CH_3$ | H | H |
| $C_2H_5$ | 6-$CH_3$ | H | H | $CH_3$ | H | H |
| $CH_3$ | 6-$CH_3$ | H | H | H | $CH_3$ | H |
| $C_2H_5$ | 6-$C_2H_5$ | H | H | H | $CH_3$ | H |
| $C_2H_5$ | 6-$CH_3$ | H | H | H | $CH_3$ | H |
| $CH_3$ | 6-$CH_3$ | H | H | H | Cl | $CH_3$ |
| $C_2H_5$ | 6-$C_2H_5$ | H | H | H | Cl | $CH_3$ |
| $C_2H_5$ | 6-$CH_3$ | H | H | H | Cl | $CH_3$ |
| $CH_3$ | 6-$CH_3$ | H | H | H | H | Cl |
| $C_2H_5$ | 6-$C_2H_5$ | H | H | H | H | Cl |
| $C_2H_5$ | 6-$CH_3$ | H | H | H | H | Cl |
| $CH_3$ | 6-$CH_3$ | H | H | H | Cl | Cl |
| $C_2H_5$ | 6-$C_2H_5$ | H | H | H | Cl | Cl |
| $C_2H_5$ | 6-$CH_3$ | H | H | H | Cl | Cl |
| $CH_3$ | 6-$CH_3$ | H | H | Cl | Cl | H |
| $C_2H_5$ | 6-$C_2H_5$ | H | H | Cl | Cl | H |
| $C_2H_5$ | 6-$CH_3$ | H | H | Cl | Cl | H |

| $R^1$ | $R^2$ | $R^3$ | $R^4$ | $R^5$ | $R^6$ | $R^7$ |
|---|---|---|---|---|---|---|
| $CH_3$ | 6-$CH_3$ | H | H | Cl | H | H |
| $C_2H_5$ | 6-$C_2H_5$ | H | H | Cl | H | H |
| $C_2H_5$ | 6-$CH_3$ | H | H | Cl | H | H |
| $CH_3$ | 6-$CH_3$ | H | H | Br | H | H |
| $C_2H_5$ | 6-$C_2H_5$ | H | H | Br | H | H |
| $C_2H_5$ | 6-$CH_3$ | H | H | Br | H | H |
| $CH_3$ | 6-$CH_3$ | H | $CH_3$ | $CH_3$ | H | H |
| $C_2H_5$ | 6-$C_2H_5$ | H | $CH_3$ | $CH_3$ | H | H |
| $C_2H_5$ | 6-$CH_3$ | H | $CH_3$ | $CH_3$ | H | H |

Die außerdem für das erfindungsgemäße Verfahren (a) als Ausgangsstoffe zu verwendenden Säurechloride oder -bromide bzw. -anhydride sind durch die Formeln (IIIa) und (IIIb) allgemein definiert. In diesen Formeln steht $R^8$ vorzugsweise für diejenigen Reste, die bereits im Zusammenhang mit der Beschreibung der erfindungsgemäßen Stoffe der Formel (I) vorzugsweise für diesen Substituenten genannt wurden.

Die Säurechloride oder -bromide bzw. -anhydride der Formeln (IIIa) und (IIIb) sind allgemein bekannte Verbindungen der organischen Chemie.

Die bei der Durchführung des erfindungsgemäßen Verfahrens (b) als Ausgangsstoffe benötigten Anilide sind durch die Formel (IV) allgemein definiert. In dieser Formel stehen $R^1$, $R^2$, $R^3$ und $R^8$ vorzugsweise für diejenigen Reste, die bereits im Zusammenhang mit der Beschreibung der erfindungsgemäßen Stoffe der Formel (I) vorzugsweise für diese Substituenten genannt wurden.

Die Anilide der Formel (IV) können in allgemein bekannter Art und Weise erhalten werden, indem man entsprechende Aniline mit einem Säurechlorid oder -bromid bzw. -anhydrid der Formeln (IIIa) und (IIIb) gemäß den Bedingungen des Verfahrens (a) in Gegenwart eines inerten organischen Lösungsmittels wie beispielsweise Toluol oder Methylenchlorid, gegebenenfalls in Gegenwart eines Säurebindemittels, wie z.B. Kaliumcarbonat oder Triethylamin, oder in Gegenwart eines Katalysators, wie z.B. Dimethylformamid, bei Temperaturen zwischen 0 und 100°C umsetzt (vergleiche auch die Herstellungsbeispiele).

Als Beispiele für die Ausgangsstoffe der Formel (IV) seien genannt:

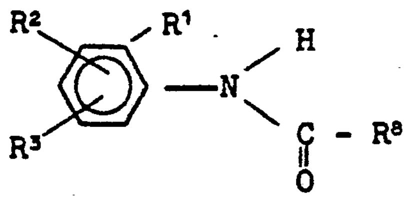

(IV)

| $R^1$ | $R^2$ | $R^3$ | $R^8$ |
|---|---|---|---|
| $CH_3$ | $6-CH_3$ | H | 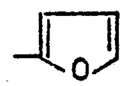 |
| $C_2H_5$ | $6-CH_3$ | H | 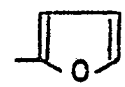 |
| $C_2H_5$ | $6-C_2H_5$ | H | 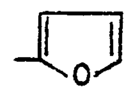 |
| $CH_3$ | $3-CH_3$ | $6-CH_3$ | 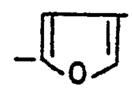 |
| Cl | $6-CH_3$ | H | 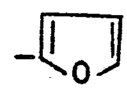 |
| $CH_3$ | $6-CH_3$ | H | $-CH_2-O-CH_3$ |
| $C_2H_5$ | $6-CH_3$ | H | $-CH_2-O-CH_3$ |
| $C_2H_5$ | $6-C_2H_5$ | H | $-CH_2-O-CH_3$ |
| $CH_3$ | $3-CH_3$ | $6-CH_3$ | $-CH_2-O-CH_3$ |
| Cl | $6-CH_3$ | H | $-CH_2-O-CH_3$ |
| $CH_3$ | $6-CH_3$ | H | $-COOCH_3$ |
| $C_2H_5$ | $6-CH_3$ | H | $-COOCH_3$ |
| $C_2H_5$ | $6-C_2H_5$ | H | $-COOCH_3$ |
| $CH_3$ | $3-CH_3$ | $6-CH_3$ | $-COOCH_3$ |
| Cl | $6-CH_3$ | H | $-COOCH_3$ |

| $R^1$ | $R^2$ | $R^3$ | $R^8$ |
|---|---|---|---|
| $CH_3$ | 6-$CH_3$ | H | $-CH_2-$ 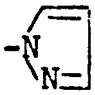 |
| $C_2H_5$ | 6-$CH_3$ | H | $-CH_2-$ 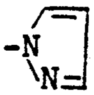 |
| $C_2H_5$ | 6-$C_2H_5$ | H | $-CH_2-$ 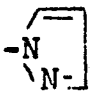 |
| $CH_3$ | 3-$CH_3$ | 6-$CH_3$ | $-CH_2-$ 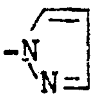 |
| Cl | 6-$CH_3$ | H | $-CH_2-$ 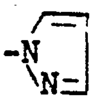 |
| $CH_3$ | 6-$CH_3$ | H | $-CH_2-$ 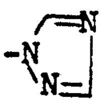 |
| $C_2H_5$ | 6-$CH_3$ | H | $-CH_2-$ 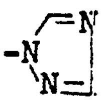 |
| $C_2H_5$ | 6-$C_2H_5$ | H | $-CH_2-$ 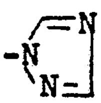 |
| $CH_3$ | 3-$CH_3$ | 6-$CH_3$ | $-CH_2-$ 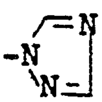 |
| Cl | 6-$CH_3$ | H | $-CH_2-$ 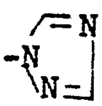 |
| $CH_3$ | 6-$CH_3$ | H | $-CH_2-$ 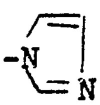 |
| $C_2H_5$ | 6-$CH_3$ | H | $-CH_2-$ 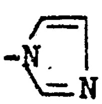 |
| $C_2H_5$ | 6-$C_2H_5$ | H | $-CH_2-$ 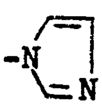 |
| $CH_3$ | 3-$CH_3$ | 6-$CH_3$ | $-CH_2-$ 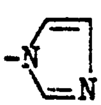 |
| Cl | 6-$CH_3$ | H | $-CH_2-$ 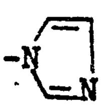 |

| $R^1$ | $R^2$ | $R^3$ | $R^8$ |
|---|---|---|---|
| $CH_3$ | 6-$CH_3$ | H | $-CH_2-O-SO_2CH_3$ |
| $C_2H_5$ | 6-$CH_3$ | H | $-CH_2-O-SO_2CH_3$ |
| $C_2H_5$ | 6-$C_2H_5$ | H | $-CH_2-O-SO_2CH_3$ |
| $CH_3$ | 3-$CH_3$ | 6-$CH_3$ | $-CH_2-O-SO_2CH_3$ |
| Cl | 6-$CH_3$ | H | $-CH_2-O-SO_2CH_3$ |
| $CH_3$ | 6-$CH_3$ | H | $-CHCl_2$ |
| $C_2H_5$ | 6-$CH_3$ | H | $-CHCl_2$ |
| $C_2H_5$ | 6-$C_2H_5$ | H | $-CHCl_2$ |
| $CH_3$ | 3-$CH_3$ | H | $-CHCl_2$ |
| Cl | 6-$CH_3$ | H | $-CHCl_2$ |
| $CH_3$ | 6-$CH_3$ | H | $-OC_2H_5$ |
| $C_2H_5$ | 6-$CH_3$ | H | $-OC_2H_5$ |
| $C_2H_5$ | 6-$C_2H_5$ | H | $-OC_2H_5$ |
| $CH_3$ | 3-$CH_3$ | 6-$CH_3$ | $-OC_2H_5$ |
| Cl | 6-$CH_3$ | H | $-OC_2H_5$ |
| $CH_3$ | 6-$CH_3$ | H | 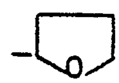 |
| $C_2H_5$ | 6-$CH_3$ | H | 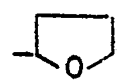 |
| $C_2H_5$ | 6-$C_2H_5$ | H | 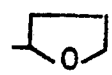 |
| $CH_3$ | 3-$CH_3$ | 6-$CH_3$ | 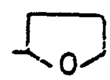 |
| Cl | 6-$CH_3$ | H | 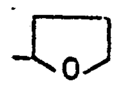 |

| $R^1$ | $R^2$ | $R^3$ | $R^8$ |
|---|---|---|---|
| $CH_3$ | 6-$CH_3$ | H | $-CH_2-O-CH_2-o-C_2H_5$ |
| $C_2H_5$ | 6-$CH_3$ | H | $-CH_2-O-CH_2-O-C_2H_5$ |
| $C_2H_5$ | 6-$C_2H_5$ | H | $-CH_2-O-CH_2-O-C_2H_5$ |
| $CH_3$ | 3-$CH_3$ | 6-$CH_3$ | $-CH_2-O-CH_2-o-C_2H_5$ |
| Cl | 6-$CH_3$ | H | $-CH_2-O-CH_2-O-C_2H_5$ |
| $CH_3$ | 6-$CH_3$ | H | $-CH_2-O-$ 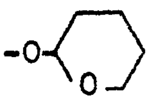 |
| $C_2H_5$ | 6-$CH_3$ | H | $-CH_2-O-$ 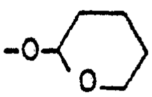 |
| $C_2H_5$ | 6-$C_2H_5$ | H | $-CH_2-O-$ 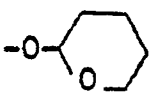 |
| $CH_3$ | 3-$CH_3$ | 6-$CH_3$ | $-CH_2-O-$ 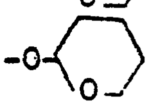 |
| Cl | 6-$CH_3$ | H | $-CH_2-O-$ 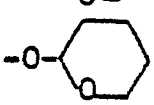 |

Die außerdem für das erfindungsgemäße Verfahren (b) als Ausgangsstoffe zu verwendenden Allylhalogenide sind durch die Formel (V) allgemein definiert. In dieser Formel stehen $R^4$ bis $R^7$ vorzugsweise für diejenigen Reste, die bereits im Zusammenhang mit der Beschreibung der erfindungsgemäßen Stoffe der Formel (I) vorzugsweise für diese Substituenten genannt wurden.

Die Allylhalogenide der Formel (V) sind allgemein bekannte Verbindungen der organischen Chemie.

Die bei der Durchführung des erfindungsgemäßen Verfahrens (c) als Ausgangsstoffe zu verwendenden Aldehyde bzw. Ketone sind durch die Formel (VI) allgemein definiert. In dieser Formel stehen $\underline{R}^1$ <u>bis</u> $\underline{R}^5$ und $\underline{R}^8$ vorzugsweise für diejenigen Reste, die bereits im Zusammenhang mit der Beschreibung der erfindungsgemäßen Stoffe der Formel (I) vorzugsweise für diese Substituenten genannt wurden.

Die Aldehyde bzw. Ketone der Formel (VI) sind noch nicht bekannt. Sie lassen sich jedoch in einfacher Weise nach mehreren Verfahren herstellen.So erhält man Aldehyde bzw. Ketone der Formel (VI), wenn man

i) Acetanilide der Formel

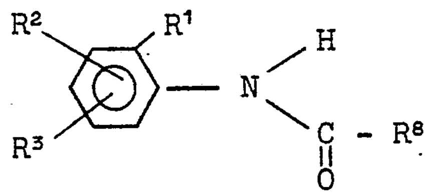

(IV)

in welcher

$R^1$, $R^2$, $R^3$ und $R^8$ die oben angegebene Bedeutung haben,

mit bekannten Keto-Derivaten der Formel

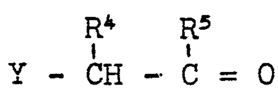

(XVI)

in welcher

$R^4$, $R^5$ und Y die oben angegebene Bedeutung haben,

in Gegenwart eines Säurebinders und gegebenenfalls in Gegenwart eines Verdünnungsmittels umsetzt, oder

j) Anilin-Derivate der Formel

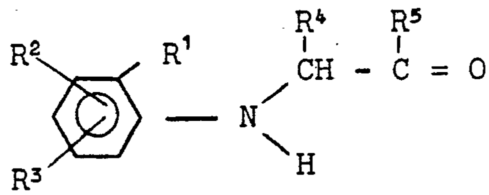

(XVII)

in welcher

$R^1$ bis $R^5$ die oben angegebene Bedeutung haben,

mit Säurechloriden oder -bromiden der Formel

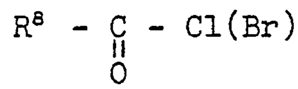

(IIIa)

in Gegenwart eines Verdünnungsmittels und gegebenenfalls in Gegenwart eines Säurebindemittels umsetzt.

Die bei der Herstellung der Aldehyde bzw. Ketone der Formel (VI) als Ausgangsstoffe benötigten Anilin-Derivate der Formel (XVII) können hergestellt werden, wenn man entsprechende Amine nach Verfahren (i) mit Keto-Derivaten der Formel (XVI) umsetzt.

Bei der Herstellung der Aldehyde bzw. Ketone der Formel (VI) nach den Verfahren (i) und (j) können als Säurebindemittel alle üblichen Säureakzeptoren verwendet werden. Vorzugsweise in Betracht kommen Alkalicarbonate, wie Kalium- oder Natriumcarbonat.

Als Verdünnungsmittel können bei den Verfahren (i) und (j) alle üblichen inerten organischen Lösungsmittel eingesetzt werden. Vorzugsweise in Betracht kommen aromatische Lösungsmittel, wie insbesondere Toluol, sowie Dimethylformamid.

Nach einer bevorzugten Ausführungsform wird die Umsetzung gemäß Verfahren (j) in einem Zweiphasensystem, wie beispielsweise wässrige Natron- oder Kalilauge/Toluol oder Methylenchlorid, gegebenenfalls unter Zusatz von 0,1-1 Mol eines Phasentransfer-Katalysators, wie beispielsweise einer Ammonium- oder Phosphoniumverbindung durchgeführt.

Die Reaktionstemperaturen können bei den Verfahren (i) und (j) innerhalb eines größeren Bereichs variiert werden. Im allgemeinen arbeitet man zwischen 0°C und 180°C, vorzugsweise zwischen 20°C und 150°C.

Bei der Umsetzung gemäß den Verfahren (i) und (j) arbeitet man vorzugsweise mit äquimolaren Mengen. Die Aufarbeitung und Isolierung der Reaktionsprodukte erfolgt nach üblichen Methoden.

Die außerdem für das erfindungsgemäße Verfahren (c) als Ausgangsstoffe zu verwendenden Phosphinalkylene sind durch die Formel (VII) allgemein definiert. In dieser Formel stehen $\underline{R}^6$ und $\underline{R}^7$ vorzugsweise für diejenigen Reste, die bereits im Zusammenhang mit der Beschreibung der erfindungsgemäßen Stoffe der Formel (I) vorzugsweise für diese Substituenten genannt wurden.

Die Phosphinalkylene der Formel (VII) sind allgemein bekannte Verbindungen der organischen Chemie.

Die bei der Durchführung der erfindungsgemäßen Verfahren (d) und (e) als Ausgangsstoffe zu verwendenden N-Propargylacetanilide sind durch die Formel (VIII) allgemein definiert. In dieser Formel stehen $\underline{R}^1$ bis $\underline{R}^4$, $\underline{R}^7$ und $\underline{R}^8$ vorzugsweise für diejenigen Reste, die bereits im Zusammenhang mit der Beschreibung der erfindungsgemäßen Stoffe der Formel (I) vorzugsweise für diese Substituenten genannt wurden.

Die N-Propargyl-acetanilide der Formel (VIII) sind noch nicht bekannt; sie sind jedoch Gegenstand einer eigenen älteren Anmeldung, die noch nicht zum Stand der Technik gehört (vergleiche die Deutsche Patentanmeldumg P 28 47 287 vom 31.10.1978). Sie können entsprechend den Verfahren (a), (b), (f) und (g) erhalten werden, indem man jeweils anstelle der Allyl-Derivate die entsprechenden Propargyl-Derivate als Ausgangsverbindungen einsetzt.

Die bei der Durchführung des erfindungsgemäßen Verfahrens (f) als Ausgangsstoffe zu verwendenden Halogenacetanilide sind durch die Formel (X) allgemein definiert. In dieser Formel stehen $\underline{R}^1$ bis $\underline{R}^7$ vorzugsweise für diejenigen Reste, die bereits im Zusammenhang mit der Beschreibung der erfindungsgemäßen Stoffe der Formel (I) vorzugsweise für diese Substituenten genannt wurden.

Die Halogenacetanilide der Formel (X) sind teilweise bekannt (vergleiche u.a. US-Patentschriften 3 442 945 und 3 475 156 sowie DE-OS 22 18 097 und DE-OS 28 05 525). Noch nicht bekannte können nach den dort beschriebenen Verfahren und entsprechend der Verfahrensvariante (a) erhalten werden, indem man N-Allylaniline der Formel (II) mit Halogenessigsäurehalogeniden umsetzt.

Die außerdem für das erfindungsgemäße Verfahren (f) als Ausgangsstoffe zu verwendenden Verbindungen sind durch die Formel (XI) allgemein definiert. In dieser Formel stehen Az und $\underline{R}^9$ vorzugsweise für diejenigen Reste, die bereits im Zusammenhang mit der Beschreibung der erfindungsgemäßen Stoffe der Formel (I) vorzugsweise für diese Substituenten genannt wurden. B steht vorzugsweise für Wasserstoff, Natrium und Kalium.

Die Verbindungen der Formel (XI) sind allgemein bekannte Verbindungen der organischen Chemie.

Die bei der Durchführung des erfindungsgemäßen Verfahrens (g) als Ausgangsstoffe zu verwendenden substituierten N-Allyl-hydroxyacetanilide sind durch die Formel (XII) allgemein definiert. In dieser Formel stehen $R^1$ bis $R^7$ vorzugsweise für diejenigen Reste, die bereits im Zusammenhang mit der Beschreibung der erfindungsgemäßen Stoffe der Formel (I) vorzugsweise für diese Substituenten genannt wurden.

Die substituierten N-Allyl-hydroxyacetanilide der Formel (XII) sind noch nicht bekannt; sie können jedoch auf allgemein bekannte Art und Weise erhalten werden, indem man Acyloxyacetanilide der Formel

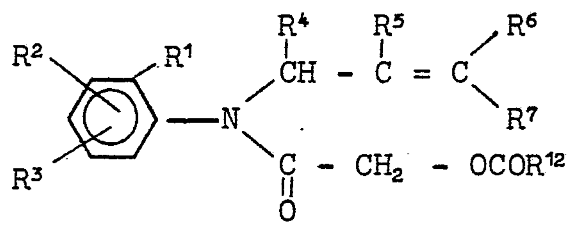 (XVIII)

in welcher

$R^1$ bis $R^7$ die oben angegebene Bedeutung haben und

$R^{12}$ für Alkyl mit 1 bis 4 Kohlenstoffatomen steht,

mit Natrium- oder Kaliumhydroxid bzw. mit einem Alkalialkoholat eines niederen Alkohols, wie z.B. Natriummethylat oder Natriumethylat, bei Temperaturen zwischen 20 und 40°C verseift und nach Ansäuren in üblicher Weise die Verbindungen der Formel (XII) isoliert.

Die Acyloxyacetanilide der Formel (XVIII) können erhalten werden, indem man z.B. bei Halogenacetaniliden der Formel (X) den reaktionsfähigen Substituenten Hal' durch Reaktion mit einer niederen Alkancarbonsäure austauscht, wobei die Säure vorzugsweise in Form ihrer Alkali- oder Erdalkalisalze eingesetzt wird; oder indem man N-Allyl-aniline der Formel (II) mit entsprechenden Acyloxyacethalogeniden umsetzt.

Die außerdem für das erfindungsgemäße Verfahren (g/α) als Ausgangsstoffe zu verwendenden Halogenide sind durch die Formel (XIII) allgemein definiert. In dieser Formel stehen $\underline{R}^{11}$ und $\underline{Hal}'$ vorzugsweise für die in der Erfindungsdefinition angegebenen Reste.

Die Halogenide der Formel (XIII) sind allgemein bekannte Verbindungen der organischen Chemie.

Das außerdem für das erfindungsgemäße Verfahren (g/ß) als Ausgangsstoff zu verwendende Dihydropyran ist ebenfalls eine bekannte Verbindung der organischen Chemie.

Die bei der Durchführung des erfindungsgemäßen Verfahrens (h) als Ausgangsstoffe zu verwendenden Hydroxyacetanilide sind durch die Formel (XV) allgemein definiert. In dieser Formel stehen $\underline{R}^1$, $\underline{R}^2$ und $\underline{R}^3$ vorzugsweise für diejenigen Reste, die bereits im Zusammenhang mit der Beschreibung der erfindungsgemäßen Stoffe der Formel (I) vorzugsweise für diese Substituenten genannt wurden.

Die Hydroxyacetanilide der Formel (XV) werden analog den Hydroxyacetaniliden der Formel (XII) erhalten.

Als Verdünnungsmittel kommen für die erfindungsgemäße Umsetzung gemäß Verfahrensvariante (a) vorzugsweise inerte organische Lösungsmittel infrage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol und Ester, wie Essigester.

Das erfindungsgemäße Verfahren (a) kann gegebenenfalls in Gegenwart von Säurebindern (Halogenwasserstoff-Akzeptoren) durchgeführt werden. Als solche können alle üblichen Säurebindemittel verwendet werden. Hierzu gehören vorzugsweise organische Basen, wie tertiäre Amine, beispielsweise Triethylamin, oder wie Pyridin, ferner anorganische Basen, wie beispielsweise Alkalihydroxide und Alkalicarbonate. Gegebenenfalls kann auch ein Katalysator, wie Dimethylformamid, verwendet werden.

Die Reaktionstemperaturen können bei der Durchführung des erfindungsgemäßen Verfahrens (a) in einem größeren Bereich variiert werden. Im allgemeinen arbeitet man zwischen 0°C und 120°C, vorzugsweise zwischen 20°C und 100°C.

Bei der Durchführung des erfindungsgemäßen Verfahrens(a) arbeitet man vorzugsweise in molaren Mengen. Die Isolierung der Verbindungen der Formel (I) erfolgt in üblicher Weise.

Als Verdünnungsmittel kommen für die erfindungsgemäße Umsetzung gemäß Verfahren (b) alle inerten organischen Lösungsmittel infrage. Hierzu gehören vorzugsweise Ether, wie Diethylether, Dioxan oder Tetrahydrofuran; aromatische Kohlenwasserstoffe,wie Benzol, Toluol oder Xylol; halogenierte Kohlenwasserstoffe, wie Methylenchlorid, Tetrachlorkohlenstoff, Chloroform oder Chlorbenzol; Ester, wie Essigester; Ketone, wie Methylisobutylketon; Nitrile, wie Acetonitril sowie Dimethylformamid oder Dimethylsulfoxid.

Die erfindungsgemäße Umsetzung gemäß Verfahren (b) wird in Gegenwart eines Säurebindemittels durchgeführt. Als solche können alle üblichen Säurebindemittel verwendet werden. Hierzu gehören vorzugsweise anorganische Basen, wie beispielsweise Alkalihydroxide und Alkalicarbonate.

Die Reaktionstemperaturen können bei der Durchführung des erfindungsgemäßen Verfahrens (b) in einem größeren Bereich variiert werden. Im allgemeinen arbeitet man zwischen -70°C und +100°C, vorzugsweise zwischen -20°C und +80°C.

Bei der Durchführung des erfindungsgemäßen Verfahrens (b) setzt man vorzugsweise auf 1 Mol Anilid der Formel (IV) 1 bis 1,5 Mol Allylhalogenid der Formel (V) ein. Die Isolierung der Verbindungen der Formel (I) erfolgt in üblicher Weise.

In einer bevorzugten Ausführungsform wird die erfindungsgemäße Umsetzung gemäß Verfahren (b) in einem Zweiphasensystem, wie beispielsweise wässrige Natron- oder Kalilauge/Toluol oder Methylenchlorid, gegebenenfalls unter Zusatz von 0,1 - 1 Mol eines Phasentransfer-Katalysators, wie beispielsweise Ammonium- oder Phosphoniumverbindungen, beispielsweise seien Benzyl-dodecyl-dimethyl-ammoniumchlorid und Triethyl-benzyl-ammoniumchlorid genannt, durchgeführt.

Als Verdünnungsmittel kommen für die erfindungsgemäße Umsetzung gemäß Verfahren (c) vorzugsweise inerte organische Lösungsmittel infrage. Hierzu gehören vorzugsweise Ether, wie Diethylether, Dioxan oder Tetrahydrofuran, aromatische Kohlenwasserstoffe, wie Benzol und Toluol; halogenierte Kohlenwasserstoffe, wie Methylenchlorid und Tetrachlorkohlenstoff; Alkohole, wie Ethanol und Methanol; sowie Dimethylformamid oder Dimethylsulfoxid.

Die Reaktionstemperaturen können bei der Durchführung des erfindungsgemäßen Verfahrens (c) in einem größeren Bereich variiert werden. Im allgemeinen arbeitet man zwischen 0°C und 100°C, vorzugsweise zwischen 20°C und 80°C.

Bei der Durchführung des erfindungsgemäßen Verfahrens (c) arbeitet man vorzugsweise in molaren Mengen. Die Isolierung der Verbindungen der Formel (I) erfolgt in üblicher Weise.

Als Verdünnungsmittel kommen für die erfindungsgemäße Umsetzung gemäß Verfahren (d) vorzugsweise polare organische Lösungsmittel infrage. Hierzu gehören vorzugsweise Alkohole, wie Methanol und Ethanol; sowie Nitrile, wie Acetonitril.

Die Umsetzung gemäß Verfahren (d) wird in Gegenwart eines Katalysators vorgenommen. Vorzugsweise werden Edelmetall-, Edelmetalloxid-(bzw. Edelmetallhydroxid-)Katalysatoren oder sogenannte 'Raney-Katalysatoren' verwendet, insbesondere Platin, Platinoxid und Nickel.

Die Reaktionstemperaturen können bei derDurchführung des erfidungsgemäßen Verfahrens (d) in einem größeren Bereich variiert werden. Im allgemeinen arbeitet man zwischen 20°C und 60°C, vorzugsweise bei 20°C bis 40°C.

Die Umsetzung gemäß Verfahren (d) kann bei Normaldruck, aber auch bei erhöhtem Druck, wie insbesondere 1 bis 2 atü, durchgeführt werden.

Bei der Durchführung des erfindungsgemäßen Verfahrens (d) setzt man vorzugsweise auf 1 Mol der Verbindungen der Formel (VIII) etwa 1 Mol Wasserstoff und 0,1 Mol Katalysator ein. Zur Isolierung der Verbindungen der Formel (I) wird vom Katalysator abfiltriert und in üblicher Weise aufgearbeitet.

Als Verdünnungsmittel kommen für die erfindungsgemäße Umsetzung gemäß Verfahren (e) vorzugsweise inerte organische Lösungsmittel infrage. Hierzu gehören vorzugsweise chlorierte Kohlenwasserstoffe, wie Tetrachlorkohlenstoff, Chloroform oder Dichlormethan; Alkohole, wie Ethanol; aromatische Kohlenwasserstoffe, wie Chlorbenzol oder Nitrobenzol; sowie Eisessig.

Die Umsetzung gemäß Verfahren (e) wird in Gegenwart eines Katalysators vorgenommen. Hierzu gehören vorzugsweise Metallhalogenide, wie z.B. Eisen-(III)-halogenid, Aluminium-halogenid, Wismut-(III)-halogenid, Antimon-(III)-halogenid, Quecksilber-(II)-halogenid und Kupfer-(I)-oder (II)-halogenid sowie Metallhalogenidgemische.

Die Umsetzung gemäß Verfahren (e) kann auch in der Gasphase sowie bei geringem Ueberdruck vorgenommen werden.

Die Reaktionstemperaturen können bei der Durchführung des erfindungsgemäßen Verfahrens (e) in einem größeren Bereich variiert werden. Im allgemeinen arbeitet man zwischen -20°C bis +250°C, vorzugsweise bei 0°C bis 200°C.

Bei der Durchführung des erfindungsgemäßen Verfahrens (e) arbeitet man vorzugsweise in molaren Mengen. Die Isolierung der Verbindungen der Formel (I) erfolgt in üblicher Weise.

Als Verdünnungsmittel kommen für die erfindungsgemäße Umsetzung gemäß Verfahren (f) vorzugsweise inerte organische Lösungsmittel infrage. Hierzu gehören vorzugsweise die beim Verfahren (a) bereits genannten Lösungsmittel.

Die Umsetzung nach Verfahren (f) kann gegebenenfalls in Gegenwart eines Säurebinders durchgeführt werden. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, Dimethylbenzylamin; oder wie Pyridin und Diazabicyclooctan. Vorzugsweise verwendet man einen Ueberschuß an Azol.

Die Reaktionstemperaturen können beim Verfahren (f) in einem größeren Bereich variiert werden. Im allgemeinen arbeitet man zwischen etwa 20°C bis 150°C, vorzugsweise bei 60°C bis 120°C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens (f) setzt man auf 1 Mol der Verbindungen der Formel (X) vorzugsweise 1 bis 2 Mol der Verbindungen der Formel (XI) und gegebenenfalls 1 bis 2 Mol Säurebinder ein. Die Isolierung der Verbindungen der Formel (I) erfolgt in üblicher Weise.

Als Verdünnungsmittel kommen für die erfindungsgemäße Umsetzung gemäß Verfahren (g) vorzugsweise inerte organische Lösungsmittel infrage. Hierzu gehören vorzugsweise die beim Verfahren (a) bereits genannten Lösungsmittel.

Die Umsetzung nach Verfahren (g/α) kann gegebenenfalls in Gegenwart eines Säurebinders durchgeführt werden. Man kann alle üblicherweise verwendbaren anorganischen oder organischen Säurebinder zugeben. Hierzu gehören vorzugsweise die beim Verfahren (a) bereits genannten Verbindungen.

Die Reaktionstemperaturen können beim Verfahren (g/α) in einem größeren Bereich variiert werden. Im allgemeinen arbeitet man zwischen 20°C und 150°C, vorzugsweise zwischen 20°C und 100°C.

Bei der Durchführung des erfindungsgemäßen Verfahrens (g/α) setzt man auf 1 Mol der Verbindungen der Formel (XII), gegebenenfalls nach Zugabe von 1 bis 2 Mol einer starken Base, wie z.B. eines Alkalihydrids, 1 Mol Halogenid der Formel (XIII) und gegebenenfalls 1 bis 2 Mol Säurebinder ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt und in üblicher Weise aufgearbeitet.

Nach einer bevorzugten Ausführungsform des Verfahrens(g/α) wird zweckmäßigerweise so verfahren, daß man von einem Hydroxyacetanilid der Formel (XII) ausgeht, letzteres in einem geeigneten inerten Lösungsmittel mittels Alkalimetall-hydrid oder -amid in das Alkalimetall-alkanolat überführt, und letzteres ohne Isolierung sofort mit einem Halogenid der Formel (XIII) umsetzt, wobei unter Austritt von Alkalihalogenid die erfindungsgemäßen Verbindungen der Formel (I) in einem Arbeitsgang erhalten werden.

In einer weiteren bevorzugten Ausführungsform wird die erfindungsgemäße Umsetzung gemäß Verfahren (g/α) in einem wässrig -organischen Zweiphasensystem durchgeführt (vergleiche hierzu die Angaben bei Verfahren (b)).

Die Umsetzung nach Verfahren (g/ß) kann gegebenenfalls in Gegenwart eines Katalysators durchgeführt werden. Hierzu verwendet man vorzugsweise Chlorwasserstoff (vgl. J.Am. Chem.Soc. 69, 2246 (1947), ibid. 70, 4187 (1948)).

Die Reaktionstemperaturen können beim Verfahren (g/ß) in einem größeren Bereich variiert werden. Im allgemeinen arbeitet man zwischen 0°C und 100°C, vorzugsweise zwischen 20°C und 60°C.

Bei der Durchführung des erfindungsgemäßen Verfahrens (g/ß) arbeitet man vorzugsweise in molaren Mengen. Die Isolierung der Verbindungen der Formel (I) erfolgt in üblicher Weise.

Als Verdünnungsmittel kommen für die erfindungsgemäße Umsetzung gemäß Verfahren (h) vorzugsweise inerte organische Lösungsmittel infrage. Hierzu gehören vorzugsweise die beim Verfahren (a) bereits genannten Lösungsmittel.

Die Umsetzung nach Verfahren (h) kann gegebenenfalls in Gegenwart eines Säurebinders durchgeführt werden. Man kann alle üblicherweise verwendbaren anorganischen oder organischen Säurebinder zugeben. Hierzu gehören vorzugsweise die beim Verfahren (a) bereits genannten Verbindungen.

Die Reaktionstemperaturen können beim Verfahren (h) in einem größeren Bereich variiert werden. Im allgemeinen arbeitet man zwischen 20°C und 120°C, vorzugsweise zwischen 20°C und 80°C.

Bei der Durchführung des erfindungsgemäßen Verfahrens (h) setzt man auf 1 Mol der Verbindungen der Formel(XV), gegebenenfalls nach Zugabe von 1 bis 2 Mol einer starken Base, wie z.B. eines Alkalihydrids, 2 bis 2,5 Mol Halogenid der Formel (V) und gegebenenfalls 2 bis 4 Mol Säurebinder ein. Die Isolierung der Endprodukte sowie bevorzugte Ausführungsformen entsprechen den Angaben beim Verfahren (g/α).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Oomyceten, z.B. gegen den Erreger der Kraut- und Braunfäule der Tomate und Kartoffel (Phytophthora infestans) eingesetzt werden. Besonders hervorzuheben ist, daß die erfindungsgemäßen Wirkstoffe nicht nur eine protektive, sondern auch eine kurativ-eradikative Wirkung entfalten. Außerdem besitzen sie systemische Eigenschaften. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beispiel A

Phytophthora-Test (Tomaten)/ Protektiv

Lösungsmittel: 4,7 Gewichtsteile Aceton
Emulgator : 0,3 Gewichtsteile Alkyl-aryl-polyglykolether
Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.
Mit der Spritzflüssigkeit bespritzt man junge Tomatenpflanzen mit 2 bis 4 Laubblättern bis zur Tropfnässe. Die Pflanzen verbleiben 24 Stunden bei 20°C und einer relativen Luftfeuchtigkeit von 70% im Gewächshaus. Anschließend werden die Tomatenpflanzen mit einer wässrigen Sporensuspension von Phytophthora infestans inokuliert. Die Pflanzen werden in eine Feuchtkammer mit einer 100%igen Luftfeuchtigkeit und einer Temperatur von 18 bis 20°C gebracht.
Nach 5 Tagen wird der Befall der Tomatenpflanzen bestimmt. Die erhaltenen Boniturwerte werden auf Prozent Befall umgerechnet. 0 % bedeutet keinen Befall, 100 % bedeutet, daß die Pflanzen vollständig befallen sind.
In diesem Test zeigen z.B. folgende Verbindungen eine sehr gute Wirkung, die derjenigen der aus dem Stand der Technik bekannten Verbindungen deutlich überlegen ist.

Verbindungen gemäß Herstellungsbeispielen 3, 1, 4, 5, 6, 7, 8, 9, 10 und 11.

**Beispiel B**

Phytophthora-Test (Tomaten)/ Systemisch

Lösungsmittel : 4,7 Gewichtsteile Aceton
Dispergiermittel: 0,3 Gewichtsteile Alkyl-aryl-polyglykolether
Wasser : 95,0 Gewichtsteile

Man vermischt die für die gewünschte Wirkstoffkonzentration in der Gießflüssigkeit nötige Wirkstoffmenge mit der angegebenen Menge des Lösungsmittels und verdünnt das Konzentrat mit der angegebenen Menge Wasser, welches die genannten Zusätze enthält.
In Einheitserde angezogene Tomatenpflanzen mit 2 bis 4 Laubblättern werden mit 10 ml der Gießflüssigkeit in der angegebenen Wirkstoffkonzentration, bezogen auf 100 ml Erde, gegossen.

Die so behandelten Pflanzen werden nach der Behandlung mit einer wässrigen Sporensuspension von Phytophthora infestans inokuliert. Die Pflanzen werden in eine Feuchtkammer mit einer Luftfeuchtigkeit von 100% und einer Temperatur von 18 bis 20°C gebracht. Nach 5 Tagen wird der Befall der Tomatenpflanzen bestimmt. Die so erhaltenen Boniturwerte werden auf Prozent Befall umgerechnet. 0 % bedeutet keinen Befall, 100 % bedeutet, daß die Pflanzen vollständig befallen sind.

In diesem Test zeigen z.B. folgende Verbindungen eine sehr gute Wirkung, die derjenigen der aus dem Stand der Technik bekannten Verbindungen deutlich überlegen ist:

Verbindungen gemäß Herstellungsbeispielen 3, 1, 4, 5, 6, 7, 8, 9, 10 und 11.

Herstellungsbeispiele

Beispiel 1

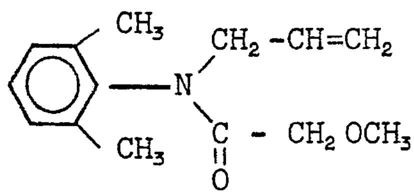

(Verfahren b)

19,3g (0,1 Mol) 2,6-Dimethyl-methoxyacetanilid werden in 200 ml Toluol gelöst und nach Zugabe von 0,5g Triethyl-benzyl-ammoniumchlorid mit 60 ml 50%iger Natronlauge versetzt. Danach werden unter schnellem Rühren 13,3g (0,11 Mol) Allylbromid zugetropft. Man läßt 5 Stunden bei 25°C rühren, trennt die Toluolphase ab, wäscht mehrmals mit Wasser, trocknet über Natriumsulfat und destilliert im Vakuum. Man erhält 15,2g (65 % der Theorie) 2,6-Dimethyl-N-allyl-methoxyacetanilid vom Siedepunkt 100-103°C/0,1mm bzw. vom Schmelzpunkt 45°C.

Herstellung des Ausgangsproduktes

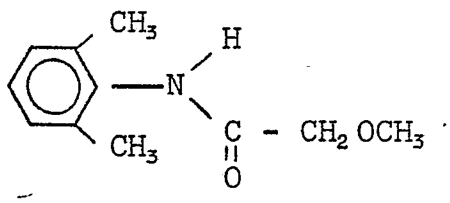

121g(1 Mol) 2,6-Dimethylanilin und 101g Triethylamin werden in 300 ml Toluol gelöst und bei ca.5°C unter Rühren tropfenweise mit 108,5g (1Mol) Methoxyessigsäurechlorid versetzt. Man läßt 4 Stunden bei 20°C rühren, filtriert, wäscht die Toluolphase zweimal mit je 50 ml Wasser, trocknet über Natriumsulfat und engt ein. Der ölige Rückstand wird im Vakuum destilliert. Man erhält 160 g (83% der Theorie) 2,6-Dimethyl-methoxyacetanilid vom Siedepunkt 134-139°C/0,1mm bzw. vom Schmelzpunkt 60-61°C.

Beispiel 2



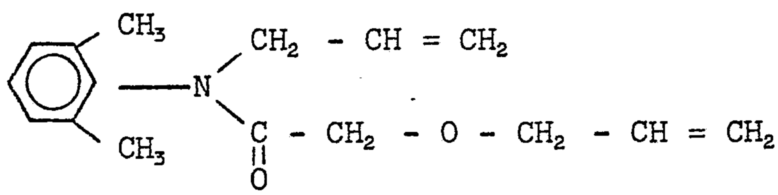

(Verfahren h)

26,6g (0,22 Mol) Allylbromid werden zu einer heftig gerührten Mischung aus 17,9g (0,1 Mol) 2,6-Dimethyl-hydroxyy-acetanilid, 1g Triethyl-benzyl-ammoniumchlorid, 300 ml Methylenchlorid und 50g 50%-iger Natronlauge getropft. Man läßt das Zweiphasensystem 12 Stunden rühren, trennt die organische Phase ab, wäscht sie mit Wasser neutral, trocknet über Natriumsulfat und engt durch Abdestillieren des Lösungsmittels im Vakuum ein. Der Rückstand wird im Hochvakuum destilliert. Man erhält 22,6g (87% der Theorie) 2,6-Dimethyl-N-allyl-allyloxyacetanilid vom Siedepunkt 115-120°C/ 0,05mm.

Entsprechend den Verfahren (a) bis (h) können die folgenden Verbindungen der allgemeinen Formel

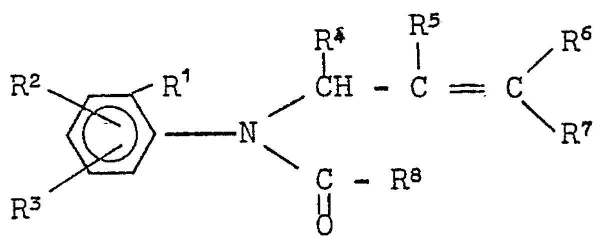

(I)

erhalten werden:

| Bsp. Nr. | $R^1$ | $R^2$ | $R^3$ | $R^4$ | $R^5$ | $R^6$ | $R^7$ | $R^8$ | Fp(°C) bzw. Kp(°C)/ mmHg-Säule |
|---|---|---|---|---|---|---|---|---|---|
| 3 | $CH_3$ | $6-CH_3$ | H | H | H | H | H | 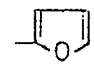 | 68-70 |
| 4 | $CH_3$ | $6-CH_3$ | H | H | H | H | H | $-CH_2-$ 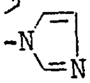 | $n_D^{20}$:1,5637 |
| 5 | $CH_3$ | $6-CH_3$ | H | H | H | Cl | $CH_3$ | $-CH_2OCH_3$ | 124-26/ 0,2 |
| 6 | $CH_3$ | $6-CH_3$ | H | H | $CH_3$ | H | H | $-CH_2OCH_3$ | 110/0,2 |
| 7 | $CH_3$ | $6-CH_3$ | H | H | H | $CH_3$ | H | $-CH_2OCH_3$ | 111/0,2 |
| 8 | $CH_3$ | $6-CH_3$ | H | H | Br | H | H | $-CH_2OCH_3$ | 128-29/ 0,2 |
| 9 | $CH_3$ | $6-CH_3$ | H | H | Cl | H | Cl | $-CH_2OCH_3$ | 128-29/ 0,2 |
| 10 | $CH_3$ | $6-CH_3$ | H | H | H | Cl | Cl | $-CH_2OCH_3$ | 46-47 |
| 11 | $CH_3$ | $6-CH_3$ | H | H | H | Cl | H | $-CH_2OCH_3$ | 128-29/ 0,1 |
| 12 | $CH_3$ | $6-CH_3$ | H | H | H | H | $CH_3$ | 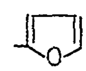 | 75-77 |
| 13 | $CH_3$ | $6-CH_3$ | H | H | $CH_3$ | H | H | 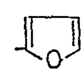 | 75-77 |
| 14 | $CH_3$ | $6-CH_3$ | H | H | H | Cl | $CH_3$ | 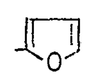 | 106-08 |
| 15 | $CH_3$ | $6-CH_3$ | H | H | $CH_3$ | H | Cl | 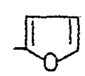 | 81-82 |
| 16 | $CH_3$ | $6-CH_3$ | H | H | H | H | Cl | 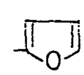 | 84-86 |
| 17 | $CH_3$ | $6-CH_3$ | H | H | H | Cl | Cl | 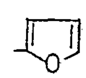 | 89-91 |
| 18 | $CH_3$ | $6-CH_3$ | H | H | Br | H | H | 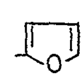 | 53-55 |
| 19 | $C_2H_5$ | $6-C_2H_5$ | H | H | H | H | H | 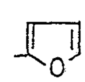 | 36-37 |
| 20 | $CH_3$ | $6-CH_3$ | H | H | $CH_3$ | H | Cl | $-CH_2OCH_3$ | 130-35/ 0,1 |
| 21 | $C_2H_5$ | $6-C_2H_5$ | H | H | H | H | H | $-CHCl_2$ | $n_D^{20}$:1,5442 |
| 22 | $CH_3$ | $6-C_2H_5$ | H | H | H | H | H | $-CHCl_2$ | $n_D^{20}$:1,5464 |

0020859



| Bsp. Nr. | $R^1$ | $R^2$ | $R^3$ | $R^4$ | $R^5$ | $R^6$ | $R^7$ | $R^8$ | Fp(°C) bzw. Kp(°C)/ mmHg-S. |
|---|---|---|---|---|---|---|---|---|---|
| 23 | $CH_3$ | $6-CH_3$ | H | H | H | H | H | $-CHCl_2$ | 66-67 |
| 24 | $CH_3$ | $6-CH_3$ | H | $-CH_2O$<br>$CH_3$ | H | H | H | $-CH_2OCH_3$ | 132-35/ 0,2 |
| 25 | $CH_3$ | $6-CH_3$ | H | $-CH_2O$<br>$CH_3$ | H | H | H | 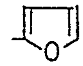 | 182/0,3 |
| 26 | $CH_3$ | $6-C_2H_5$ | H | $-CH_2O$<br>$CH_3$ | H | H | H | $-CH_2OCH_3$ | 150-54/ 0,1 |
| 27 | $CH_3$ | $6-C_2H_5$ | H | $-CH_2O$<br>$CH_3$ | H | H | H | 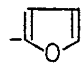 | 67-72 |
| 28 | $CH_3$ | $6-C_2H_5$ | H | $-CH_2O$<br>$C_2H_5$ | H | H | H | $-CH_2OCH_3$ | $n_D^{20}$:1,5157 |
| 29 | $CH_3$ | $6-CH_3$ | H | H | H | H | H | $-CH_2SCH_3$ | 150/0,2 |

| Bsp. Nr. | $R^1$ | $R^2$ | $R^3$ | $R^4$ | $R^5$ | $R^6$ | $R^7$ | $R^8$ | Fp($^{o}$C) bzw. Kp ($^{o}$C)/ mmHg-S. |
|---|---|---|---|---|---|---|---|---|---|
| 30 | $CH_3$ | 6-$CH_3$ | H | $CH_3$ | H | H | H | $CH_2OCH_3$ | $n_D^{21}$: 1,5172 |
| 31 | $CH_3$ | 6-$CH_3$ | H | $CH_3$ | H | H | H | 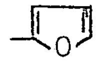 | 70 - 73 |
| 32 | $CH_3$ | 6-$CH_3$ | H | $CH_3$ | H | H | H | $CH_2Cl$ | $n_D^{26}$: 1,5339 |
| 33 | $CH_3$ | 6-$CH_3$ | H | $CH_3$ | H | H | H | CO-$OCH_3$ | $n_D^{26}$: 1,5069 |
| 34 | $CH_3$ | 6-$CH_3$ | H | $CH_3$ | H | H | H | $CHCl_2$ | $n_D^{23}$: 1,5373 |
| 35 | $CH_3$ | 6-$CH_3$ | H | $CH_3$ | H | H | H | 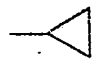 | $n_D^{23}$: 1,5229 |
| 36 | $CH_3$ | 6-$CH_3$ | H | $CH_3$ | H | H | H | $CH_2$-O-CO-$CH_3$ | $n_D^{22,5}$: 1,5080 |
| 37 | $CH_3$ | 6-$CH_3$ | H | $CH_3$ | H | H | H | $C_3H_7$-n | $n_D^{22,5}$: 1,5058 |
| 38 | $CH_3$ | 6-$CH_3$ | H | $CH_3$ | H | H | H | $CH_2$- 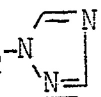 | 90 - 91 |
| 39 | $CH_3$ | 6-$CH_3$ | H | $CH_3$ | H | H | H | $CH_2$- 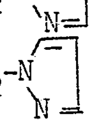 | 57 - 58 |
| 40 | $CH_3$ | 6-$CH_3$ | H | $CH_3$ | H | H | H | $CH_2$-OH | $n_D^{24}$: 1,5290 |
| 41 | $CH_3$ | 6-$CH_3$ | H | $CH_3$ | H | H | H | $CH_2$-O-$SO_2$-$CH_3$ | $n_D^{26}$: 1,5184 |
| 42 | $CH_3$ | 6-$CH_3$ | H | $CH_3$ | H | H | H | $CH_2$- 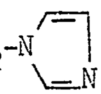 | $n_D^{22}$: 1,5415 |

Patentansprüche

1. Substituierte N-Allyl-acetanilide der allgemeinen Formel

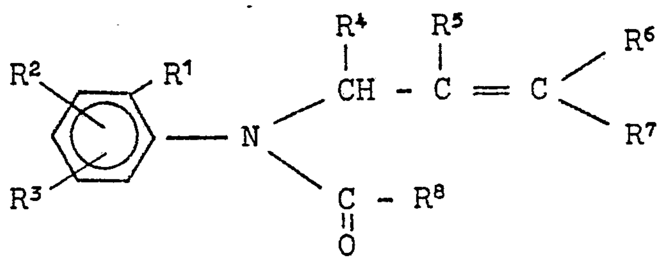

(I)

in welcher

$R^1$ für Wasserstoff, Alkyl, Halogen, Alkoxyalkyl oder Alkylthioalkyl steht,

$R^2$ für Wasserstoff, Alkyl, Alkoxyalkyl oder Alkylthioalkyl steht,

$R^3$ für Wasserstoff, Alkyl, Alkoxyalkyl oder Alkylthioalkyl steht,

$R^4$ für Wasserstoff, Alkyl oder Alkoxyalkyl steht,

$R^5$ für Wasserstoff, Alkyl, Halogen oder gegebenenfalls substituiertes Phenyl steht,

$R^6$ für Wasserstoff, Alkyl, Halogen oder gegebenenfalls substituiertes Phenyl steht,

$R^7$ für Wasserstoff, Alkyl, Halogen oder gegebenenfalls substituiertes Phenyl steht,

$R^8$ für Furyl, Tetrahydrofuryl, Thiophenyl, Tetrahydrothiophenyl; gegebenenfalls durch Alkyl substituiertes Isoxazolyl; gegebenenfalls durch Cyano oder Thiocyano substituiertes Alkyl, Alkenyl und Alkinyl; Dihalogenalkyl; sowie die Gruppierungen

$-CH_2-Az$, $-CH_2-OR^9$, $-CH_2-SR^9$, $-OR^9$, $-SR^9$, $-CH_2-OSO_2R^9$, $-COOR^9$ und $-CH_2-O-$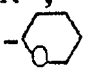
steht, wobei

$R^9$ für gegebenenfalls substituiertes Alkyl, Alkenyl, Alkinyl oder Alkoxyalkyl steht und

Az für Pyrazol-1-yl, 1,2,4-Triazol-1-yl und Imidazol-1-yl steht.

2. Verfahren zur Herstellung von substituierten N-Allyl-acetaniliden der Formel (I), dadurch gekennzeichnet, daß man

a) N-Allyl-aniline der Formel

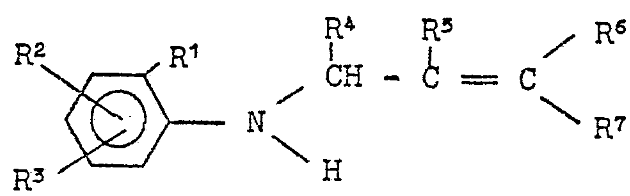
(II)

in welcher

$R^1$ bis $R^7$ die oben angegebene Bedeutung haben,

mit Säurechloriden oder -bromiden bzw. -anhydriden der Formeln

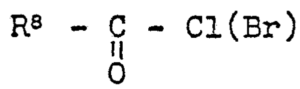 (IIIa)

bzw.

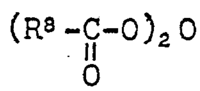 (IIIb)

in welchen

$R^8$ die oben angegebene Bedeutung hat,

in Gegenwart eines Verdünnungsmittels und gegebenenfalls in Gegenwart eines Säurebindemittels umsetzt, oder

b) Anilide der Formel

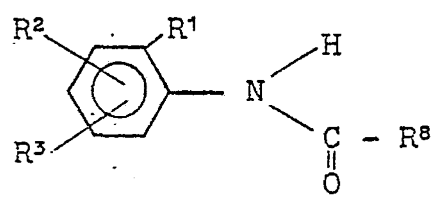 (IV)

in welcher

$R^1$, $R^2$, $R^3$ und $R^8$ die oben angegebene Bedeutung haben

mit Allylhalogeniden der Formel

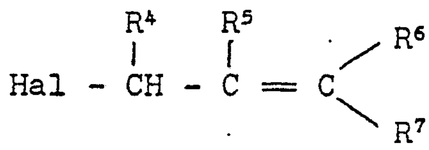

(V)

in welcher

$R^4$ bis $R^7$ die oben angegebene Bedeutung haben und

Hal für Chlor oder Brom steht,

in Gegenwart eines Säurebinders und gegebenenfalls in Gegenwart eines organischen Verdünnungsmittels oder in einem wässrig-organischen Zweiphasensystem in Gegenwart eines Phasentransferkatalysators umsetzt, oder

c) Aldehyde bzw. Ketone der Formel

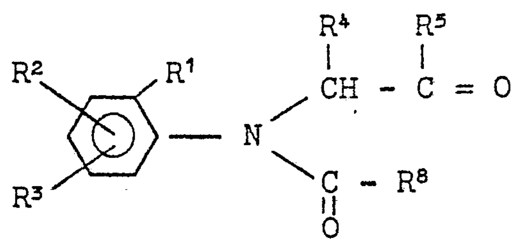

(VI)

in welcher

$R^1$ bis $R^5$ und $R^8$ die oben angegebene Bedeutung haben,

mit Phosphinalkylenen der Formel

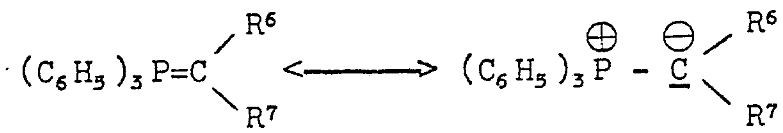 (VII)

in welcher

$R^6$ und $R^7$ die oben angegebene Bedeutung haben,

gegebenenfalls in Gegenwart eines Verdünnungsmittels umsetzt,

oder einzelne Verbindungen der Formel (I) auch dadurch erhält, daß man

d) N-Propargyl-acetanilide der Formel

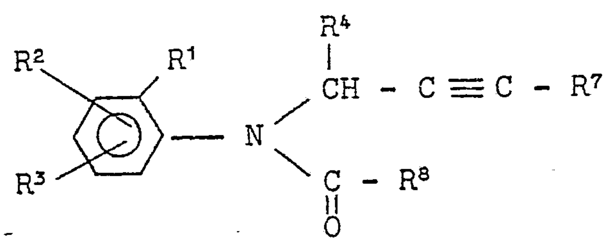 (VIII)

in welcher

$R^1$ bis $R^4$,
$R^7$ und $R^8$ die oben angegebene Bedeutung haben,

mit Wasserstoff in Gegenwart eines Katalysators und gegebenenfalls in Gegenwart eines polaren Lösungsmittels selektiv hydriert, oder

e) N-Propargyl-acetanilide der Formel

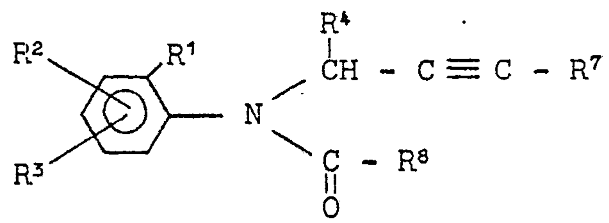

(VIII)

in welcher

$R^1$ bis $R^4$,
$R^7$ und $R^8$ die oben angegebene Bedeutung haben,

mit Halogenen bzw. Hydrohalogeniden der Formel

$$X - Y \qquad (IX)$$

in welcher

X für Wasserstoff oder Halogen steht und
Y für Halogen steht,

in Gegenwart eines Katalysators und gegebenenfalls in Gegenwart eines Lösungsmittels umsetzt, oder

f) Halogenacetanilide der Formel

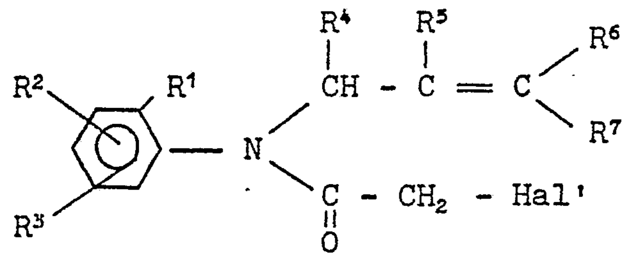

(X)

in welcher

$R^1$ bis $R^7$ die oben angegebene Bedeutung haben und

Hal' für Chlor, Brom oder Jod steht

mit Verbindungen der Formel

$$B - R^{10} \qquad (XI)$$

in welcher

B für Wasserstoff oder ein Alkalimetall steht und

$R^{10}$ für Az und die Gruppierung $-OR^9$ oder $-SR_9$ steht,

in Gegenwart eines Verdünnungsmittels und gegebenenfalls in Gegenwart eines Säurebinders umsetzt, oder

g) substituierte N-Allyl-hydroxyacetanilide der Formel

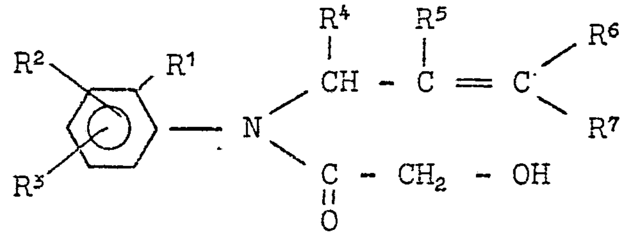

(XII)

in welcher

$R^1$ bis $R^7$ die oben angegebene Bedeutung haben,

(α) gegebenenfalls nach Aktivierung mittels Alkalimetall mit Halogeniden der Formel

$R^{11}$ - Hal' (XIII)

in welcher

Hal' die oben angegebene Bedeutung hat und

$R^{11}$ für den Rest $R^9$ sowie die Gruppe $-SO_2-R^9$ steht, wobei $R^9$ die oben angegebene Bedeutung hat,

in Gegenwart eines organischen Verdünnungsmittels und gegebenenfalls in Gegenwart eines Säurebinders oder in einem wässrig-organischen Zweiphasensystem in Gegenwart eines Phasentransferkatalysators umsetzt, oder

(ß) mit Dihydropyran der Formel

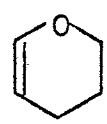

(XIV)

in Gegenwart eines Verdünnungsmittels und gegebenenfalls in Gegenwart eines Katalysators umsetzt, oder

h) Hydroxyacetanilide der Formel

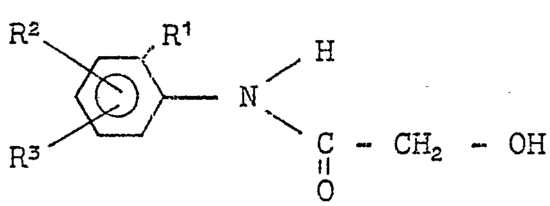

(XV)

in welcher

$R^1$ bis $R^3$ die oben angegebene Bedeutung haben,

gegebenenfalls nach Aktivierung mittels Alkalimetall mit Allylhalogeniden der Formel

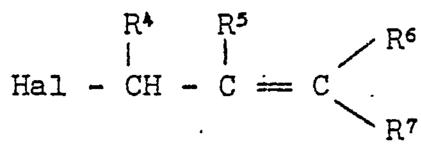

(V)

in welcher

$R^4$ bis $R^7$
und Hal die oben angegebene Bedeutung haben,

gegebenenfalls in Gegenwart eines Säurebinders und in Gegenwart eines organischen Verdünnungsmittels oder in einem wässrig-organischen Zweiphasensystem in Gegenwart eines Phasentransferkatalysators umsetzt.

3. Fungizide Mittel, gekennzeichnet durch einen Gehalt an mindestens einem substituierten N-Allyl-acetanilid der Formel (I).

4. Verfahren zur Bekämpfung von Pilzen, dadurch gekennzeichnet, daß man substituierte N-Allyl-acetanilide der Formel (I) auf Pilze oder ihren Lebensraum einwirken läßt.

5. Verwendung von substituierten N-Allyl-acetaniliden der Formel (I) zur Bekämpfung von Pilzen.

6. Verfahren zur Herstellung von fungiziden Mitteln, dadurch gekennzeichnet, daß man substituierte N-Allyl-acetanilide der Formel (I) mit Streckmitteln und/oder oberflächenaktiven Mitteln vermischt.

Europäisches Patentamt

# EUROPÄISCHER RECHERCHENBERICHT

0020859

Nummer der Anmeldung

EP 80 10 1339

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US - A - 3 048 619 (B.O. PRAY)<br>* Patentschrift *<br>-- | 1,2 |
| PX | EP - A - 0 005 591 (ICI)<br>* Patentansprüche *<br>-- | 1,2 |
| X | CHEMICAL ABSTRACTS, vol. 89, nr. 9, 28.August 1978, Seite 405, Zusammenfassung 75356s<br>Columbus, Ohio, USA<br>MELIKYAN T.R et al. "Studies on amines and ammonium compounds. CXXXIX. Intramolecular cyclization of N-allylcinnamamides and N-allylfurancarboxylic acid amides".<br>& Arm. Khim. Zh., 1977, 30(12), 981-6.<br>* Zusammenfassung *<br>-- | 1 |
| | US - A - 4 021 224 (F. PALLOS et al.)<br>* Zusammenfassung *<br>-- | 1,2 |
| E | EP - A - 0 010 673 (BAYER)<br>* Patentansprüche *<br>---- | 1,3,4,5,6 |

**KLASSIFIKATION DER ANMELDUNG (Int. Cl.³)**

C 07 C 103/365
103/375
C 07 D 307/68
C 07 D 233/56
C 07 C 149/23
103/737
C 07 D 231/12
249/08
A 01 N 43/00
47/00
C 07 D 261/18
A 01 N 37/22
A 01 N 41/00

**RECHERCHIERTE SACHGEBIETE (Int. Cl.³)**

C 07 C 103/365
103/375
C 07 D 307/68
C 07 D 233/56
C 07 C 149/23
103/737
C 07 D 231/12
249/08
A 01 N 43/00
47/00
C 07 D 261/18
A 01 N 37/22
41/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 11-09-1980 | CREMERS |